(19) Europäisches Patentamt
European Patent Office
Office européen des brevets

(11) EP 1 736 544 A1

(12) **EUROPEAN PATENT APPLICATION**
published in accordance with Art. 158(3) EPC

(43) Date of publication:
**27.12.2006 Bulletin 2006/52**

(21) Application number: **05721227.6**

(22) Date of filing: **15.03.2005**

(51) Int Cl.:
***C12N 15/11*** *(2006.01)* ***A61K 45/00*** *(2006.01)*
***A61P 25/18*** *(2006.01)* ***A61P 25/22*** *(2006.01)*
***A61P 25/24*** *(2006.01)* ***C12N 1/15*** *(2006.01)*
***C12N 1/19*** *(2006.01)* ***C12N 1/21*** *(2006.01)*
***C12N 5/10*** *(2006.01)* ***C12Q 1/02*** *(2006.01)*
***C12Q 1/68*** *(2006.01)* ***G01N 33/15*** *(2006.01)*
***G01N 33/50*** *(2006.01)*

(86) International application number:
**PCT/JP2005/005077**

(87) International publication number:
**WO 2005/108574 (17.11.2005 Gazette 2005/46)**

(84) Designated Contracting States:
**AT BE BG CH CY CZ DE DK EE ES FI FR GB GR HU IE IS IT LI LT LU MC NL PL PT RO SE SI SK TR**

(30) Priority: **15.03.2004 JP 2004072244**

(71) Applicant: **Sumitomo Chemical Company, Limited**
**Tokyo 104-8260 (JP)**

(72) Inventors:
- **TAKAHASHI, Yasuhiko**
  **5610802 (JP)**
- **OEDA, Kenji**
  **Kyoto-shi,**
  **Kyoto 6038147 (JP)**

(74) Representative: **Vossius & Partner**
**Siebertstrasse 4**
**81675 München (DE)**

(54) **Gm1 PROMOTER AND USE THEREOF**

(57) The present invention relates to a polynucleotide comprising a nucleotide sequence of a promoter region of a gene encoding α subunit Gm1 of trimeric G-protein, more specifically, said polynucleotide, wherein the nucleotide sequence of a promoter region is, for example, any of the following nucleotide sequences (1) and (2):
(1) the nucleotide sequence of SEQ ID NO: 1, and
(2) the nucleotide sequence of the nucleotide numbers 603 to 3871 in the nucleotide sequence of SEQ ID NO: 1 and the like.

EP 1 736 544 A1

## Description

Technical Field

The present invention relates to a promoter of a gene encoding α subunit Gm1 of trimeric G-protein, and use thereof.

Background Art

G-protein serves as a transmitter which transports into a cell a signal of stimulus received by a G-protein coupled receptor (hereinafter, referred to as GPCR in some cases) that is a seven transmembrane receptor. G-protein is composed of α, β and γ subunits. In inactivated G-protein in which GDP is bound to the α subunit, when a receptor agonist such as hormones, neurotransmitters and the like binds to GPCR, GDP is released from the α subunit, and GTP binds instead. Activated G-protein in which GTP is bound to the α subunit is released from GPCR and dissociated into a GTP-bound α subunit and βγ subunits. Activated G-protein controls a variety of cellular functions by promoting or suppressing its target effector such as adenylate cyclase, $Ca^{2+}$ channel, $K^+$ channel, phospholipase Cβ and the like. GTP which has been bound to an α subunit of activated G-protein is converted into GDP by the effect of GTPase possessed by the α subunit, returning to inactivated form.

Thus far, various kinds of G-proteins have been identified in cells of mammals. These G-proteins show respective specific tissue distributions. There are known G-proteins distributed, for example, in nerve cells, olfactory cells, visual cells, gustatory cells, lung cells, kidney cells, liver cells and the like. G-proteins play important roles in hormone reception, neurotransmission and cell proliferation and differentiation, and the like by correlating with a cellular signal transduction system in such specific tissues.

It has been clarified that various diseases are induced by loss of normal functions of α subunits of G-proteins. For example, constant activation of G-protein α subunit Gs induces diseases such as pituitary tumor, thyroid tumor, McCune-Albright syndrome and the like. Loss of functions of G-protein α subunit Gs induces pseudo-hypoparathyroidism. Constant activation of G-protein α subunit Gi2b induces pituitary tumor, adrenal cortex tumor or uterus tumor (for example, G protein mutations in endocrine diseases, European Journal of Endocrinology (2001) vol. 145, 545-559).

DISCLOSURE OF THE INVENTION

The present invention provides a polynucleotide comprising a nucleotide sequence having an ability of controlling transcription of a gene encoding α subunit Gm1 of trimeric G-protein. This polynucleotide can be utilized in a method for searching a compound as an active ingredient of medicines for treatment, improvement or prevention (namely, therapeutic drugs, improving drugs or preventing drugs) of diseases ascribable to abnormality in expression amount of α subunit Gm1 of trimeric G-protein or abnormality in signal transduction through a promoter of a gene encoding this subunit, and the like.

More specifically, the present invention provides

1. A polynucleotide comprising a nucleotide sequence of a promoter region of a gene encoding α subunit Gm1 of trimeric G-protein (hereinafter, referred to as polynucleotide of the present invention in some cases);
2. The polynucleotide according to the item 1, wherein the nucleotide sequence of a promoter region is any of the following nucleotide sequences (1) to (4):

    (1) the nucleotide sequence of SEQ ID NO: 1,
    (2) the nucleotide sequence of the nucleotide numbers 603 to 3871 in the nucleotide sequence of SEQ ID NO: 1,
    (3) a nucleotide sequence of (1) or (2) with deletion, substitution or addition of one or more nucleotides, said nucleotide sequence having an ability of controlling the transcription of a gene encoding α subunit Gm1 of trimeric G-protein, and
    (4) a nucleotide sequence having an ability of controlling the transcription of a gene encoding α subunit Gm1 of trimeric G-protein, and being complementary to a nucleotide sequence of a polynucleotide, wherein said polynucleotide hybridizes under a stringent condition to a polynucleotide comprising the nucleotide sequence of (1) or (2);

3. A plasmid comprising the polynucleotide of the item 1 or 2 (hereinafter, referred to as plasmid of the present invention in some cases);
4. A plasmid comprising the polynucleotide of the item 1 or 2, wherein at the downstream (3' side) of said polynucleotide, said plasmid contains a polynucleotide of which transcription is controlled by said polynucleotide;

5. A plasmid comprising the polynucleotide of the item 1 or 2, wherein at the downstream (3' side) of said polynucleotide, said plasmid contains a reporter gene of which transcription is controlled by said polynucleotide;
6. A transformed cell in which the polynucleotide of the item 1 or 2 is introduced;
7. A transformed cell in which the plasmid of the item 3 or 4 is introduced;
8. A transformed cell in which the plasmid of the item 5 is introduced(hereinafter, referred to as transformed cell of the present invention together with the transformed cell of the item 6 or 7, in some cases);
9. A method for searching a signal transduction controlling substance through a promoter of a gene encoding α subunit Gm1 of trimeric G-protein, comprising

(1) a first step of contacting the transformed cell of the item 8 with a test substance,
(2) a second step of monitoring the expression amount of a reporter gene or an index value correlated therewith, after the first step,
(3) a third step of evaluating an ability of the above-mentioned substance to control signal transduction through a promoter of a gene encoding α subunit Gm1 of trimeric G-protein, based on a change in the expression amount or index value correlated therewith monitored in the second step, and
(4) a fourth step of selecting a substance having an ability to control signal transduction through a promoter of a gene encoding α subunit Gm1 of trimeric G-protein, based on the signal transduction controlling ability evaluated in the third step (hereinafter, referred to as searching method of the present invention in some cases);

10. A method for evaluating an ability of a substance to control signal transduction through a promoter of a gene encoding α subunit Gm1 of trimeric G-protein, comprising

(1) a first step of contacting the transformed cell of the item 8 with a test substance,
(2) a second step of monitoring the expression amount of a reporter gene or an index value correlated therewith, after the first step, and
(3) a third step of evaluating an ability of the above-mentioned substance to control signal transduction through a promoter of a gene encoding α subunit Gm1 of trimeric G-protein, based on a change in the expression amount or index value correlated therewith monitored in the second step (hereinafter, referred to as evaluation method of the present invention in some cases);

11. A method for searching a substance which binds to the polynucleotide of the item 1, comprising

(1) a first step of contacting the polynucleotide of the item 1 with a test substance,
(2) a second step of checking the presence or absence of formation of a complex of the polynucleotide with the test substance, after the first step, and
(3) a third step of selecting a substance which binds to the polynucleotide, based on the analysis result, obtained in the second step, of the presence or absence of formation of a complex (hereinafter, referred to as binding substance searching method of the present invention in some cases);

12. A method for purifying a substance which binds to the polynucleotide of the item 1, comprising

(1) a first step of contacting the polynucleotide of the item 1 with a sample to form a complex of the polynucleotide with a substance, wherein said substance is contained in the sample and binds to the polynucleotide, and
(2) a second step of isolating the substance which binds to the polynucleotide, from a formed complex, after the first step (hereinafter, referred to as purification method of the present invention in some cases);

13. A kit for screening a signal transduction controlling substance through a promoter of a gene encoding α subunit Gm1 of trimeric G-protein, comprising the transformed cell of the item 8 and a reagent for measuring the expression amount of a reporter gene or an index value correlated therewith (hereinafter, referred to as kit of the present invention in some cases); and
14. A medicine for neurological disorder and/or psychiatric diseases comprising as an active ingredient a compound having an ability to control signal transduction through a promoter of a gene encoding α subunit Gm1 of trimeric G-protein, obtained by the searching method of the item 9 or 11, or a pharmaceutically acceptable salt thereof, wherein the active ingredient is formulated in a pharmaceutically acceptable carrier

(hereinafter, referred to as medicine of the present invention in some cases); and the like.

## BRIEF DESCRIPTION OF THE DRAWINGS

Fig. 1 is a view showing a transcription-activating ability of the Gm1 promoter of the present invention.
Fig. 2 is a view showing expression of α subunit Gm1 of trimeric G-protein in brain tissues (cerebral cortex, hippocampus, cerebellum).
Fig. 3 is a view showing expression of α subunit Gm1 of trimeric G-protein in brain tissues of a patient with psychiatric disease.

## BEST MODES FOR CARRYING OUT THE INVENTION

The present invention will be illustrated in detail below.

The genetic engineering techniques to be used in the present invention can be carried out according to conventional methods described in, for example, "Molecular Cloning: A Laboratory Manual 2nd edition" (1989), Cold Spring Harbor Laboratory Press and, D.M. Glover, DNA Cloning, published by IRL, 1985, and the like.

Alpha (α) subunit Gm1 of trimeric G-protein is highly expressed in neuron of cerebral cortex, hippocampus and cerebellum. For example, in brains of patients with neurological disorder and/or psychiatric diseases (schizophrenia, manic-depressive psychosis, depression and anxiety), the expression of the Gm1 is lowered. Lowering of the expression amount of G-protein causes decline in function of transmitting a signal from outside of a cell into a cell. This suggests that lowering of the expression amount of Gm1 is correlated with symptoms of these neurological disorder and/or psychiatric diseases (schizophrenia, manic-depressive psychosis, depression and anxiety).

The polynucleotide of the present invention is a polynucleotide comprising a nucleotide sequence of a region (about 3.8 kb) located upstream of a cording region in a gene encoding α subunit Gm1 of trimeric G-protein, and is a kind of regulatory gene so-called promoter. The nucleotide sequence of the polynucleotide contains a nucleotide sequence necessary for promoting transcription and is, for example, a site where a RNA polymerase containing σ factor binds and initiates the transcription of an operon. Further, the nucleotide sequence of the polynucleotide may contain, for example, a promoter element sequence which is controlled cell type-specifically or tissue-specifically, or a promoter element sequence sufficient for causing promoter-dependent gene expression induced by an exogenous signal or factor (for example, transcription activation protein).

The transformed cell in which a plasmid is introduced into a mammal cell, wherein said plasmid comprises the polynucleotide of the present invention and contains at the downstream (3' side) of the polynucleotide a reporter gene of which transcription is controlled by the polynucleotide, shows higher expression amount of the reporter gene as compared with a transformed cell in which a plasmid containing a reporter gene and not containing the polynucleotide is introduced. That is, the polynucleotide of the present invention has an ability of controlling the transcription of a gene located downstream thereof, for example, in a mammal cell, thus, it is a polynucleotide comprising a nucleotide sequence having an ability of controlling the transcription of a gene encoding α subunit Gm1 of trimeric G-protein.

Specific examples of the polynucleotide of the present invention include polynucleotides having nucleotide sequences of any of the following sequences (1) to (4), and the like.

(1) The nucleotide sequence of SEQ ID NO: 1.
(2) The nucleotide sequence of the nucleotide numbers 603 to 3871 in the nucleotide sequence of SEQ ID NO: 1 (said sequence is a nucleotide sequence which corresponds to the nucleotide sequence of SEQ ID NO: 2).
(3) A nucleotide sequence of the above (1) or (2) with deletion, substitution or addition of one or more nucleotides, said nucleotide sequence having an ability of controlling the transcription of a gene encoding α subunit Gm1 of trimeric G-protein (hereinafter, said ability is referred to as the present transcription controlling ability in some cases).
(4) A nucleotide sequence having an ability of controlling the transcription of a gene encoding α subunit Gm1 of trimeric G-protein, and being complementary to a nucleotide sequence of a polynucleotide, wherein said polynucleotide hybridizes under a stringent condition to a polynucleotide comprising the nucleotide sequence of the above (1) or (2).

Examples of the nucleotide sequence of the polynucleotide of the present invention include also nucleotide sequences of polynucleotides obtained by partial deletion of nucleotides (for example, prepared by excising using a suitable restriction enzyme) while maintaining an ability of the polynucleotide of the present invention to control the transcription of a gene encoding α subunit Gm1 of trimeric G-protein. Specific examples of such nucleotide sequences include the nucleotide sequence of the nucleotide numbers 100 to 3871 in the nucleotide sequence of SEQ ID NO: 1, the nucleotide sequence of the nucleotide numbers 200 to 3871 in the nucleotide sequence of SEQ ID NO: 1, the nucleotide sequence of the nucleotide numbers 300 to 3871 in the nucleotide sequence of SEQ ID NO: 1, the nucleotide sequence of the nucleotide numbers 400 to 3871 in the nucleotide sequence of SEQ ID NO: 1, the nucleotide sequence of the nucleotide numbers 500 to 3871 in the nucleotide sequence of SEQ ID NO: 1, and the like. Further, the nucleotide sequence of any of the above-mentioned nucleotide sequences with deletion, substitution or addition of one or more nucleotides, said nucleotide sequence having an ability of controlling the transcription of a gene encoding α subunit Gm1 of trimeric G-protein; the nucleotide sequence having an ability of controlling the transcription of a gene encoding α subunit Gm1 of trimeric G-protein, and being complementary to a nucleotide sequence of a polynucleotide, wherein said polynucleotide hybridizes under a stringent condition to a polynucleotide comprising any of the above-mentioned nucleotide sequences; and the like, are also mentioned.

Such polynucleotides of the present invention may be prepared according to conventional genetic engineering methods, for example, methods described in "Takaaki Tamura (published by yodosha), Shin Tensha Seigyo no Mechanism (2000)" pages 33 to 40, "Shintaro Nomura, Toshiki Watanabe ed. (published by shujunsha), Datsu Isotope Jikken Protocol (1998)", and the like. The ability of controlling the transcription of a gene encoding α subunit Gm1 of trimeric G-protein can be confirmed by a method described below, for example, by reporter gene assay using a transformed cell in which a plasmid is introduced, wherein said plasmid comprises the polynucleotide and contains a reporter gene at the downstream (3' side) of the polynucleotide.

In the present invention, "polynucleotide" (including oligonucleotide) includes also polynucleotides comprising a nucleotide sequence complementary to a nucleotide sequence of the polynucleotide. "Polynucleotide" includes both of DNA and RNA unless otherwise stated.

"DNA" includes single-stranded DNA comprising its nucleotide sequence, and additionally, single-stranded DNA complementary thereto and double-stranded DNA. Further, "DNA" includes cDNA, genomic DNA and synthesized DNA unless otherwise stated. "RNA" includes total RNA, mRNA, rRNA and synthesized RNA unless otherwise stated.

In the present invention, "alteration not causing loss of transcription activity" means, for example, alteration which can be carried out on a region with low homology to various transcription control sequences (namely, nucleotide sequences having an ability of controlling transcription) identified previously, and the like. In "nucleotide sequence with deletion, substitution or addition of one or more nucleotides" containing such alteration, an index for the kind and number of nucleotides which can be deleted, substituted or added without causing loss of an ability of controlling the transcription of a gene encoding α subunit Gm1 of trimeric G-protein can be evaluated and confirmed by a method described below.

Such alteration may also be prepared by artificial mutagenesis. Specifically, such alteration can be achieved by random mutagenesis using a method described in A. Greener, M. Callahan, Strategies (1994) 7, 32-34 and the like, and can be achieved by site-directed mutagenesis using a gapped duplex method described in W. Kramer, et al. , Nucleic Acids Research (1984) 12, 9441 or W. Kramer, H. J. Frits, Methods in Enzymology (1987) 154, 350 and the like, or a Kunkel method described in T. A. Kunkel, Proc. Of Natl. Acad. Sci. U.S.A. (1985) 82, 488 or T. A. Kunkel, et al. , Methods in Enzymology (1987) 154, 367 and the like. Alternatively, such alteration can be obtained by preparing chimeric DNA with substitution of a part of a polynucleotide of other promoter for one or several partial nucleotide sequences of a polynucleotide comprising the nucleotide sequence of SEQ ID NO: 1 and the like, where, for example, a method described in S. Henikoff, et al., Gene (1984) 28, 351, C. Yanisch-Perron, et al., Gene (1985) 33, 103 and the like can be used.

In the instant application, "polynucleotide which hybridizes under a stringent condition" denotes a polynucleotide shown below. That is, it means a polynucleotide (1) which forms a polynucleotide-polynucleotide hybrid by hybridizing at temperature condition of 65°C under high ion concentration [for example, 6xSSC (containing 900 mM sodium chloride and 90 mM sodium citrate) and the like are mentioned] and (2) in which the hybrid can be maintained even after washing for 30 minutes at temperature condition of 65°C under low ion concentration [for example, 0.1×SSC (containing 15 mM sodium chloride and 1.5 mM sodium citrate) and the like are used].

Nucleotide sequences of such polynucleotides include nucleotide sequences derived from other organisms (for example, human and rat) which corresponds to a mouse-derived nucleotide sequence. Such nucleotide sequences can be selected, for example, by blast search provided by NCBI (www.ncbi.nlm.nih.gov/BLAST/) and the like. Specifically, a nucleotide sequence showing high homology can be searched from a data base of genomic sequence of other organisms, by blast search provided by NCBI using, as a query sequence, a nucleotide sequence near a translation initiation codon of Gm1 gene, which comprises the nucleotide sequence of SEQ ID NO: 3. By selecting a polynucleotide comprising a nucleotide sequence upstream of the translation initiation site of a nucleotide sequence selected by this search, a polynucleotide comprising a nucleotide sequence containing a promoter region of corresponding other organisms can be selected. The ability of the selected polynucleotide to control the transcription of a gene encoding α subunit Gm1 of trimeric G-protein can be confirmed by a method described later.

As the method for preparing the polynucleotide of the present invention, for example, a chemical synthesis method, PCR, hybridization method and the like are mentioned.

In the case of preparation using a chemical synthesis method, DNA automatic synthesizing machines, for example, DNA synthesizing machine model 380A (manufactured by ABI) and the like can be used.

Next, the method of preparing the polynucleotide of the present invention using PCR will be illustrated. A genomic library to be used as a template can be prepared from tissue of mammals such as human, mouse, rat and the like according to a method described in, for example, "Molecular Cloning: A Laboratory Manual 2nd edition" (1989), Cold Spring Harbor Laboratory Press and the like. Further, commercially available genomic DNAs such as human genomic DNA (manufactured by Clontech) and the like, and commercially available genomic libraries such as human Genome-Walker kit (Clontech) and the like, can be used. Then, PCR is performed using primers corresponding to a promoter to be amplified, and in the case of preparing the polynucleotide of the present invention comprising the nucleotide sequence of SEQ ID NO: 1, using, for example, a primer comprising the nucleotide sequence of the nucleotide numbers 1 to 20 in the nucleotide sequence of SEQ ID NO: 1, and a primer comprising a nucleotide sequence complimentary to the nucleotide sequence of the nucleotide numbers 3851 to 3871 in SEQ ID NO: 1. Such primers can be appropriately designed based on the nucleotide sequence of SEQ ID NO: 1. A restriction enzyme recognition sequence and the like may be added to its 5' end. DNA amplified as described above can be cloned to a vector according to a conventional method described in "Molecular Cloning: A Laboratory Manual 2nd edition" (1989), Cold Spring Harbor Laboratory Press, "Current Protocols In Molecular Biology" (1987), John Wiley & Sons, Inc. ISBN0-471-50338-X and the like. Specifically, cloning can be performed using a plasmid vector contained in TA cloning kit manufactured by Invitrogen, or plasmid vector such as pBluescript II manufactured by Stratagene, and the like. The nucleotide sequence of the cloned polynucleotide can be analyzed by a dideoxy terminating method described in F. Sanger, S. Nicklen, A.R. Coulson, Proceedings of National Academy of Science U.S.A. (1977), 74, 5463-5467, and the like.

Next, the method of preparing the polynucleotide of the present invention using a hybridization method will be illustrated.

First, DNA to be used as a probe is labeled. As the DNA to be used as a probe, mentioned are polynucleotides comprising at least a part of the nucleotide sequence of the polynucleotide of the present invention to be prepared, for example, a polynucleotide comprising the nucleotide sequence of the nucleotide numbers 603 to 3871 or partial continuous nucleotide sequence thereof in the nucleotide sequence of SEQ ID NO: 1, and having a chain length of 20 nucleotides or more and 1000 nucleotides or less, a polynucleotide comprising a nucleotide sequence of the above-mentioned polynucleotide with deletion, substitution or addition of one or several nucleotides, a polynucleotide which hybridizes under a stringent condition to the above-mentioned polynucleotide, and the like. Specifically mentioned are DNA comprising a nucleotide sequence of the nucleotide numbers 100 to 3871 in the nucleotide sequence of SEQ ID NO: 1, DNA comprising a nucleotide sequence of the nucleotide numbers 200 to 3871 in the nucleotide sequence of SEQ ID NO: 1, DNA comprising a nucleotide sequence of the nucleotide numbers 300 to 3871 in the nucleotide sequence of SEQ ID NO: 1, DNA comprising a nucleotide sequence of the nucleotide numbers 400 to 3871 in the nucleotide sequence of SEQ ID NO: 1, DNA comprising a nucleotide sequence of the nucleotide numbers 500 to 3871 in the nucleotide sequence of SEQ ID NO: 1, and the like.

The above-mentioned DNA to be used as a probe can be obtained by the conventional polynucleotide preparation methods described above as "the method of preparing the polynucleotide of the present invention" such as, for example, a chemical synthesis method, PCR, hybridization method and the like.

For labeling the above-mentioned DNA to be used as a probe with a radioisotope, for example, Random Labelling Kit and the like manufactured by Boehringer, Takara Shuzo Co., Ltd., and the like can be used, and labeling can also be carried out by performing a PCR reaction using as a template the above-mentioned DNA to be used as a probe, replacing dCTP in a conventional PCR reaction composition by [$\alpha$-$^{32}$P]dCTP. In the case of labeling of DNA to be used as a probe with a fluorescent dye or enzyme, there can be used, for example, ECF Direct Nucleic Acid Labelling and Detection System (manufactured by Amersham Pharmacia Biotech) or Alkphos Direct DAN labeling and detection kit (manufactured by Amersham Pharmacia Biotech) and the like.

As the DNA library to which a probe is hybridized, for example, genomic DNA libraries can be used derived from animals indlucing Rodentia such as mouse, rat and the like, and Primate such as human and the like.

As the DNA library, commercially available genomic DNA libraries can be used, and alternatively, genomic DNA libraries are produced using Gigapack packaging Extracts (Stratagene) and the like in in vitro packaging, using $\lambda$ vectors such as, for example, $\lambda$ FIX II, $\lambda$ EMBL3, $\lambda$ EMBL4, $\lambda$ DASH II and the like manufactured by Stratagene, according to a conventioinal library production method described in "Molecular Cloning: A Laboratory Manual 2nd edition" (1989), Cold Spring Harbor Laboratory Press, "Current Protocols In Molecular Biology" (1987), John Wiley & Sons, Inc. ISBNO-471-50338-X and the like, and these libraries can be used.

The hybridization method includes colony hybridization and plaque hybridization, and the method may be selected according to the kind of a vector used in preparation of a library. For example, when the library to be used is a library constructed using plasmid vectors, colony hybridization can be carried out. Specifically, first, DNA of a library is introduced into a host microorganism to obtain transformed cells, the resultant transformed cells are diluted and spread on an agar medium, and cultured at 37°C until appearance of a colony. When the library to be used is a library constructed using phage vectors, plaque hybridization can be carried out. Specifically, first, a host microorganism and a phage of the library are mixed under infectable conditions, then, further mixed with a soft agar medium, and this mixture is spread on an agar medium. Thereafter, culturing is performed at 37°C until appearance of a plaque. More specifically, a phage library of about $9.0\times10^5$ pfu is spread, at a density of 0.1 to 1.0 pfu per 1 $mm^2$ of agar medium, on NZY agar medium, and cultured at 37°C for 6 to 10 hours, according to a method described in, for example, Molecular Cloning 2nd edition (J. Sambrook, E.F. Frisch, T. Maniatis, Cold Spring Harbor Laboratory Press 1989) 2.60 to 2.65, and the like.

Next, in any of the above-mentioned hybridization methods, a membrane filter is placed on the surface of an agar medium on which the above-mentioned culturing has been effected, and a phage or transformed cell containing a plasmid are transferred onto the membrane filter. This membrane filter is treated with an alkali, then, is treated to neutralize, then, is treated to fix DNA on the filter. More specifically, in the case of plaque hybridization for example, a nitrocellulose filter or nylon filter and the like, for example, Hybond-$N^+$ (manufactured by Amersham Pharmacia Biotech) is placed on the above-mentioned agar medium, and left for about 1 minute, to make phage particles to be adsorbed on the membrane filter, according to a conventional method described in Cloning and Sequence: Plant Biotechnology Experiment Manual (Watanabe, Sugiura ed., Noson Bunka sha, 1989) and the like. Next, this filter is immersed in an alkali solution (1.5 M sodium chloride, 0.5 N sodium hydroxide) for about 3 minutes to lyse phage particles, thereby releasing phage DNA on the filter, then, immersed in a neutralization solution (1.5 M sodium chloride, 0.5 M Tris hydrochloric acid, pH 7.5) for about 5 minutes. This filter is washed with a washing solution (300 mM sodium chloride, 30 mM sodium citrate, 200 mM Tris hydrochloric acid) for about 5 minutes, then, for example, baked at about 80°C for about 90 minutes, to fix phage DNA on the filter. Hybridization is performed using thus prepared filter and the above-mentioned probe. Reagents and temperature conditions in hybridization can be determined according to a method described in, for example, D.M. Glover, "DNA cloning, a practical approach" IRL PRESS (1985) ISBN 0-947946-18-7, Cloning and Sequence: Plant Biotechnology Experiment Manual (Watanabe, Sugiura ed., Noson Bunka sha, 1989), or Molecular Cloning 2nd edition (J. Sambrook, E.F. Frisch, T. Maniatis, Cold Spring Harbor Laboratory Press 1989), and the like. For example, a pre-hybridization solution containing 450 to 900 mM sodium chloride and 45 to 90 mM sodium citrate, containing sodium dodecylsulfate (SDS) in a concentration of 0.1 to 1.0% (W/V), and containing denatured non-specific DNA in a concentration of 0 to 200 μg/mL, and depending on cases, which may contain albumin, ficoll, polyvinylpyrrolidone and the like each in a concentration of 0 to 0.2% (W/V), preferably, a pre-hybridization solution containing 900 mM sodium chloride, 90 mM sodium citrate, 1% (W/V) SDS, and 100 μg/mL denatured Calf-thymus DNA, is prepared at a proportion of 50 to 200 μL per 1 $cm^2$ of the filter produced as described above, and the filter is immersed in the solution and incubated at 42 to 68°C for 1 to 4 hours, preferably, at 45°C for 2 hours. Then, for example, a hybridization solution containing 450 to 900 mM sodium chloride and 45 to 90 mM sodium citrate, containing SDS in a concentration of 0.1 to 1.0% (W/V), and containing denatured non-specific DNA in a concentration of 0 to 200 μg/mL, and depending on cases, which may contain albumin, ficoll, polyvinylpyrrolidone and the like each in a concentration of 0 to 0.2% (W/V), preferably, a hybridization solution containing 900 mM sodium chloride, 90 mM sodium citrate, 1% (W/V) SDS, and 100 μg/mL denatured Calf-thymus DNA, is mixed with a probe prepared by the above-mentioned method (for example, a $^{32}P$-labeled probe in an amount corresponding to $1.0 \times 10^4$ to $2.0 \times 10^6$ cpm per 1 $cm^2$ of a filter) to give a mixture, the mixture being prepared at a proportion of 50 to 200 μL per 1 $cm^2$ of the filter, and the filter is immersed in the mixed solution and incubated at 42 to 68°C for 4 to 20 hours, preferably, at 45°C for 16 hours, to accomplish a hybridization reaction. After this hybridization reaction, the filter is taken out, and washed for 10 to 60 minutes once to 4 times, preferably for 15 minutes twice with a washing solution of 42 to 68°C containing 15 to 300 mM sodium chloride, 1.5 to 30 mM sodium citrate and 0.1 to 1.0% (W/V) SDS and the like, preferably, a washing solution of 55°C containing 300 mM sodium chloride, 30 mM sodium citrate and 1% (W/V) SDS. Further, the filter is rinsed slightly with 2×SSC solution (containing 300 mM sodium chloride, and 30 mM sodium citrate) before drying. This filter is, for example, subjected to autoradiography and the like to detect the position of a probe on the filter, to detect the position on the filter of DNA to be hybridized to the used probe. A clone located at a position corresponding to the position of the detected DNA on the filter is identified on an agar medium and picked up, thus, the clone comprising the DNA can be isolated. Specifically, for example, the filter is exposed to an imaging plate (Fuji Photo Film Co., Ltd.) for 4 hours, then, the plate is analyzed using an imaging analyzer BAS2000 (Fuji Photo Film Co., Ltd.), to detect a signal. Of the agar medium used for filter preparation, a portion corresponding to the position at which a signal has been detected is cut out by about 5 mm square, and this is immersed in about 500 μL of SM buffer (containing 50 mM Tris-hydrochloric acid pH 7.5, 0.1 M sodium chloride, 7 mM magnesium sulf ate, and 0.01% (W/V) gelatin) for 2 to 16 hours, preferably for 3 hours to elute phage particles. The resulting phage particle eluate is spread on an agar medium, and cultured at 37°C for 6 to 10 hours, according to a method described in Molecular Cloning 2nd edition (J. Sambrook, E.F. Frisch, T. Maniatis, Cold Spring Harbor Laboratory Press 1989) 2.60 to 2.65. Using this agar medium, phage DNA is fixed to a filter by the same method as described above, and hybridization is performed using this filter and the above-mentioned probe. Of the agar medium used for filter production, phage particles are eluted from a portion corresponding to the position at which a signal has been detected, and spread on an agar medium, and a filter is made by the same method as described above, and hybridization is performed. By repeating such identification and purification of phage clones, a phage clone containing DNA comprising a nucleotide sequence which hybridizes to the used probe is obtained.

DNA contained in a clone obtained by performing screening by hybridization as described above may be sub-cloned to a plasmid vector which allow easy preparation or analysis of DNA, for example, commercially available pUC18, pUC19, pBLUESCRIPT KS+, pBLUESCRIPT KS- and the like, to prepare plasmid DNA, and its nucleotide sequence can be determined using a dideoxy terminating method described in F. Sanger, S. Nicklen, A.R. Coulson, Proceedings of National Academy of Science U.S.A. (1977), 74, 5463-5467, and the like. Preparation of a sample to be used for nucleotide sequence analysis can be carried out, for example, according to a primer extension method described in Molecular Cloning 2nd edition (J. Sambrook, E.F. Frisch, T. Maniatis, Cold Spring Harbor Laboratory Press 1989) 13. 15, and the like. Further, a phage clone is amplified on a NZYM liquid medium according to a method described in Molecular Cloning 2nd edition (J. Sambrook, E.F. Frisch, T. Maniatis, Cold Spring Harbor Laboratory Press 1989) 2.60 to 2.65 and the like to prepare phage solution, and phage clone DNA is extracted from this phage solution using, for example, Lambda-TRAPPLUS DNA Isolation Kit (manufactured by Clontech) and the like, and a sample for nucleotide sequence analysis is prepared by, for example, the above-mentioned primer extension method using the extracted DNA as a template, and its nucleotide sequence can also be analyzed. An ability of thus produced polynucleotide to control the transcription of a gene encoding $\alpha$ subunit Gm1 of trimeric G-protein can be confirmed by a method described later.

The plasmid into which the polynucleotide of the present invention (here, the polynucleotide of the present invention means DNA) is to be inserted in preparation of the plasmid of the present invention may be a plasmid capable of functioning in a desired cell, and may also contain a marker gene (for example, kanamycin-resistance gene, hygromycin-resistance gene, neomycin-resistance gene and the like) for selecting the cell into which the plasmid has been introduced. Further, in the above-mentioned plasmid, if a gene insertion site is further present at a position where the polynucleotide of the present invention and a desired gene are operably linked on a plasmid, for example, downstream of a site where the polynucleotide of the present invention is to be inserted, it can be preferably used for construction of a plasmid for expressing a desired gene in a cell. Here, the gene insertion site is, for example, a nucleotide sequence which can be specifically recognized and digested by a restriction enzyme conventionally used in a genetic engineering method, and preferably a restriction enzyme recognition sequence solely present on a plasmid comprising the polynucleotide of the present invention. Specific examples of this plasmid include pBR322 (Boehringer Mannheim), pUC-based plasmids such as pUC12, pUC118, pUC119 (Takara Shuzo Co. , Ltd.) and the like, pBluescript and the like when E. coli is used as a host, and pUB110, pC194 and the like when Bacillus is used as a host. When yeast is used as a host, Yip5, Yep24 and the like are mentioned. When an insect cell is used as a host, AcNPV and the like are mentioned. When an animal cell is used as a host, pUC18, pUC19 and the like are mentioned.

Insertion of the polynucleotide of the present invention into the above-mentioned plasmid can be carried out according to conventional methods, for example, a method described in "Molecular Cloning: A Laboratory Manual 2nd edition" (1989), Cold Spring Harbor Laboratory Press, "Current Protocols In Molecular Biology" (1987), John Wiley & Sons, Inc. ISBN0-471-50338-X and the like.

Among the plasmids of the present invention, the plasmids to be inserted into a transformed cell used in the searching method of the present invention include, for example, a plasmid containing, at the downstream (3' side) of the polynucleotide of the present invention, a polynucleotide of which transcription is controlled by the polynucleotide of the present invention, more specifically, a plasmid containing the polynucleotide of the present invention and containing, at the downstream (3' side) of the polynucleotide, a reporter gene of which transcription is controlled by the polynucleotide of the present invention, and the like. When the reporter gene is a luciferase gene, the plasmid of the present invention containing a luciferase gene under control of the polynucleotide of the present invention can be preferably used in measuring the ability of controlling the transcription of the polynucleotide of the present invention contained in the plasmid. Such a plasmid can be produced using commercially available plasmids containing a luciferase gene, for example, plasmids such as pGL3-basic (Promega), PicaGene Basic Vector (Toyo Ink) and the like. Specifically, the plasmid of the present invention containing a luciferase gene under control of the polynucleotide of the present invention can be produced by operably inserting the polynucleotide of the present invention into a gene insertion site of pGL3-basic by a conventional method.

The method of preparing the transformed cell of the present invention (namely, a transformed cell in which the polynucleotide or plasmid of the present invention is introduced) will be illustrated below.

As a host cell into which the polynucleotide of the present invention or the plasmid of the present invention is to be introduced, bacteria such as E. coli (for example, K12), Bacillus (for example, MI114) and the like, yeast (for example, AH22), cells such as plant cells, animal cells and the like are mentioned, and cells containing therein the polynucleotide of the present invention or the plasmid of the present invention may be permissible. As the host cell for preparing the transformed cell of the present invention used in the searching method of the present invention, preferably mentioned are animal cells (for example, PC-12 cell, Neuro-2a cell, IMR-32 cell, COS-7 cell, Vero cell, CHO cell, ES cell and the like), particularly preferably mentioned are neurons. The transformed cell of the present invention in which the host cell is an ES cell includes transformed cells obtained by differentiation of ES cells in which the polynucleotide or plasmid of the present invention is introduced, and transformed cells obtained, after differentiation of ES cells, by introducing the polynucleotide of the present invention or the plasmid of the present invention into the differentiated cells. The transformed cell of the present invention includes also cells present in animal individuals (namely, transformed animal) produced by developing from these transformed cells.

As the method of introducing the polynucleotide of the present invention or the plasmid of the present invention into a host cell, introduction methods according to cells can be applied, and for example, a calcium phosphate method, electric introduction method, DEAE dextran method, micelle formation method and the like can be applied for animal cells. Specifically, mentioned as the calcium phosphate method are methods described in Grimm, S. et al., Proc. Natl. Acad. Sci. USA, 93, 10923-10927 and the like, and mentioned as the electric introduction method and DEAE dextran method are methods described in Ting, A.T. et al., EMBO J., 15, 6189-6196 and the like, and mentioned as the micelle formation method are methods described in Hawkins, C.K. et al., Proc. Natl. Acad. Sci. USA, 93, 13786-13790 and the like. In the micelle formation method, it is also possible to use commercially available reagents such as, for example, lipofectamine (manufactured by Gibco BRL), fugene (Boegringer Mannheim) and the like.

Transformed cells can be selected by utilizing, for example, a selection marker gene introduced into a host cell simultaneously with the polynucleotide of the present invention or the plasmid of the present invention, a selection marker gene contained in the plasmid of the present invention, and the like, and culturing cells subjected to a treatment of introduction of the polynucleotide of the present invention or the plasmid of the present invention, on a medium under selection conditions according to the selection marker gene.

The present invention provides a method containing a step of contacting the transformed cell of the present invention with a test substance. Namely, it is a method of searching a signal transduction controlling substance through a promoter of $\alpha$ subunit Gm1 of trimeric G-protein, containing

(1) a first step of contacting with a test substance a transformed cell in which a plasmid is introduced, wherein said plasmid comprises the polynucleotide of the present invention and contains a reporter gene at the downstream (3' side) of the polynucleotide,
(2) a second step of monitoring the expression amount of a reporter gene or an index value correlated therewith, after the first step,
(3) a third step of evaluating an ability of the above-mentioned substance to control signal transduction through a promoter of a gene encoding $\alpha$ subunit Gm1 of trimeric G-protein, based on a change in the expression amount or index value correlated therewith monitored in the second step, and
(4) a fourth step of selecting a substance having an ability to control signal transduction through a promoter of a gene encoding $\alpha$ subunit Gm1 of trimeric G-protein, based on the signal transduction controlling ability evaluated in the third step (namely, searching method of the present invention). This searching method is a method of searching a substance having an ability of controlling signal transduction through a promoter of a gene encoding $\alpha$ subunit Gm1 of trimeric G-protein, using a what is called reporter gene assay.

As the cell to be used in the searching method of the present invention, the transformed cells of the present invention using a mammal cell as a host cell are preferable, and particularly preferable are transformed cells of the present invention using, as a host cell, known mammal animal cells such as, for example, PC-12 cell, Neuro-2a cell, IMR-32 cell, Vero cell, Hela cell, CV1 cell, COS1 cell, CHO cell, ES cell and the like. The transformed cells may be prepared as described above.

The concentration of a test substance to be contacted in the above-mentioned first step with a transformed cell in which a plasmid is introduced, wherein said plasmid comprises the polynucleotide of the present invention and contains a reporter gene at the downstream (3' side) of the polynucleotide, may be usually about 0.001 $\mu$M to about 100 $\mu$M, preferably about 0.01 $\mu$M to about 10 $\mu$M, more preferably about 0.1 $\mu$M to about 10 $\mu$M. The time for contacting the cell with a test substance is usually about 1 hour or more and about 5 days, and preferably several hours to about 4 days. Preferably, it is 18 hours or more and about 60 hours, and more preferably from 24 hours to about 40 hours.

Contact of the cell with a test substance may be carried out while culturing under conditions wherein the cell can grow. For example, in the case of the transformed cell of the present invention using a mammal cell as a host, culturing can be performed in commercially available media such as D-MEM, OPTI-MEM, RPMI 1640 medium (manufactured by Gibco-BRL) and the like appropriately containing added serum derived from a mammal such as bovine fetal serum and the like. Culturing may be carried out usually at about 30°C to 40°C in the presence of about 2 % (V/V) to 10 % (V/V) carbon dioxide, and more preferably carried out at about 35°C to 37°C in the presence of about 4% (V/V) to 6% (V/V) carbon dioxide.

The number of the cell in contacting with a test substance may be usually about $1 \times 10^4$/well to about $1 \times 10^5$/well, preferably about $5 \times 10^4$/well to about $8 \times 10^4$ /well, in the case of use of for example a 96-well plate.

In the above-mentioned searching method, the method of "monitoring the expression amount of a reporter gene or an index value correlated therewith" may be any method providing it can measure continuously or discontinuously with time the expression amount of a reporter gene in the above-mentioned transformed cell or an index value correlated therewith. For example, methods for detecting the expression product of a reporter gene, that is, mRNA corresponding to the gene or a protein encoded by the gene (specifically, Northern blotting, Western blotting and the like) can be used. When an enzyme gene is used as the reporter gene, a method for measuring the activity of the enzyme, and the like may be used.

More specifically, the amount of mRNA corresponding to a reporter gene can be measured by, for example, a Northern blot method using a polynucleotide comprising a part of the reporter gene, RT-PCR and the like. The amount of a protein corresponding to a reporter gene can be measured using an antibody to a protein produced by the reporter gene.

When an enzyme gene is used as the reporter gene, the activity of the produced enzyme may be measured as "index value correlated with the expression amount of a reporter gene". As the reporter gene which can be used, those easily measuring the activity of the expressed and produced protein such as, for example, a luciferase gene, secretory alkaliphosphatase (SEAP) gene, chloramphenicol acetyltransferase gene, β-galactosidase gene and the like are preferably mentioned. For the expression level of a luciferase gene, luciferin (manufactured by, for example, Toyo Ink K.K.) as a luminescence substrate may be added to cell lysate and luminescence due to decomposition of the substrate may be measured using luminometer, liquid scintillation counter or top counter and the like. The expression level of an alkaliphosphatase gene can be measured using, for example, Lumi-Phos530 (manufactured by Wako Pure Chemical Industries, Ltd.). The expression level of a chloramphenicol acetyltransferase gene can be measured using FAST CAT Chloramphenicol Acetyltransferase Assay Kit (manufactured by Wako Pure Chemical Industries, Ltd.). The expression level of a β-galactosidase gene can be measured using Aurora Gal-XE (manufactured by Wako Pure Chemical Industries, Ltd.).

In the case of emission of fluorescence and the like from a protein encoded by the gene such as GFP (Green Fluorescent Protein) gene as the reporter gene, the amount of fluorescence emitted from the produced protein may be measured.

When contact of the cell with a test substance are carried out as described above followed by measuring the present transcription controlling ability of the polynucleotide of the present invention in the cell and an increase in the transcription controlling ability as compared with a control is detected (for example, in the case where a change of about 30% increase, preferably about 50% increase, further preferably about 100% increase is shown) or a decrease is detected (for example, in the case where a change of about 30% decrease, preferably about 50% decrease, further preferably about 100% decrease is shown), this test substance can be selected as a substance for acting on the promoter to regulate the transcription controlling ability, that is, as a signal transduction controlling substance through a promoter of a gene encoding α subunit Gm1 of trimeric G-protein.

The kind of the test substance is not particularly restricted. Examples thereof include proteins, peptides, non-peptidic compounds (carbohydrate, lipid and the like), organic low molecular weight compounds, inorganic low molecular weight compounds, fermentation products, cell extracts, plant extracts, animal tissue extracts and the like.

The transcription controlling ability of the test substance may also be evaluated by comparing the expression amount of a reporter gene operably linked to the polynucleotide of the present invention (hereinafter, referred to as measured value 1) in a cell after contacting with a test substance, with the above-mentioned expression amount in a cell not contacted with the test substance (hereinafter, referred to as measured value 2). In this case, it is recommendable that this transcription controlling ability is obtained as transcription controlling ratio according to the following formula using the measured value.

$$\text{Transcription controlling ratio (\%)} = \{(\text{measured value 1} - \text{measured value 2})/\text{measured valued 2}\}\times 100$$

Substances of which transcription controlling ratio representing the transcription controlling ability of a test substance shows a statistically significant value, specifically preferably, substances showing a ratio of 30% or more, more preferably 50% or more are selected as the substance of the transcription controlling ability.

Further, in the above-mentioned searching method, the transcription controlling ability of the above-mentioned substance may be evaluated based on a difference obtained by comparing changes of the expression amount of a reporter gene in a section using different two or more substances each independently as a test substance. Substances having the transcription controlling ability can also be selected based on the transcription controlling ability thus evaluated.

Furthermore, it may also be permissible that at least one substance among the above-mentioned different two or more substances is prepared as a substance not having the transcription controlling ability (it may be also, for example, a solvent, test system solution as back ground, and the like) and the transcription controlling ability of other test substance is evaluated. The transcription controlling ability of other test substance may also be evaluated, while utilizing the transcription controlling ability of at least one substance among the above-mentioned different two or more substances as a standard.

The kit of the present invention is a kit comprising a test cell containing a recombinant reporter vector comprising the polynucleotide of the present invention (here, DNA), and a reagent for measuring reporter activity. The kit of the present invention may further contain a control cell in which a plasmid not comprising the polynucleotide of the present invention (here, DNA) is introduced. Such a control cell can be used as a negative control. This kit can be used in the searching method of the present invention described above.

The substance selected by the searching method as described above can control the activity of the polynucleotide of the present invention, thus, it can control the expression in a cell of a gene under control of the polynucleotide of the present invention. Therefore, this substance or its pharmaceutically acceptable salt may also be utilized in the form of transcription controlling agent containing them as an active ingredient and wherein the active ingredient is formulated in a pharmaceutically acceptable carrier. This substance is useful as, for example, a medicine for diseases ascribable to excess expression or under expression of a translation product of the gene. This substance can also be utilized as a transcription controlling agent in investigating an action in a neuron and an influence on diseases (particularly, neurological disorder and/or psychiatric diseases) ascribable to abnormality of the expression amount of Gm1 or to abnormality of signal transduction through a promoter of a Gm1 gene, of desired genes, for example, genes of which correlation with neurological disorder and/or psychiatric diseases is presumed and the like, said genes being linked under control of the polynucleotide of the present invention.

Next, the method for searching a substance which binds to the polynucleotide of the present invention will be illustrated below.

The method for searching a substance which binds to the polynucleotide of the present invention comprises a first step of contacting the polynucleotide of the present invention with a test substance, a second step of checking the presence or absence of formation of a complex of the polynucleotide with the test substance, after the first step, and a third step of selecting a substance which binds to the polynucleotide, based on the analysis result, obtained in the second step, of the presence or absence of formation of a complex.

The polynucleotide to be used in the first step of contacting the polynucleotide of the present invention with a test substance is preferable in that when the polynucleotide of the present invention is labeled with a radioisotope or fluorescent dye compound using, for example, a commercially available DNA labeling kit, detection of a complex of the polynucleotide and test substance becomes easy. For labeling the polynucleotide of the present invention with a radioisotope, for example, commercially available Random Labelling Kit and the like can be used. The polynucleotide can also be labeled by performing a PCR reaction using the polynucleotide of the present invention as a template while replacing dCTP by [$\alpha$-$^{32}$P]dCTP in a usual PCR reaction composition. When the polynucleotide of the present invention is labeled with a fluorescent dye, for example, ECF Direct Nucleic Acid Labelling and Detection System, and the like can be used.

The polynucleotide of the present invention and a test substance are contacted at about 4°C to about 37°C, preferably about 20°C to about 30°C for about 5 minutes to about 60 minutes, preferably about 10 minutes to about 30 minutes in a suitable buffer, for example, a buffer such as Tris, Hepes, MES and the like, preferably in a Hepes buffer. The concentration of the test substance may be usually about 0.1 $\mu$M to about 1000 $\mu$M, and preferably 1 $\mu$M to 100 $\mu$M. The amount of the polynucleotide may be usually about 1 fmol to about 10 fmol, and preferably 2 fmol to 7 fmol.

As the method of checking the presence or absence of formation of a complex of the polynucleotide with a test substance, mentioned are a gel shift method, foot print method, BIACORE method and the like. When the test substance is a compound of relatively high molecular weight (for example, protein, DNA and the like), a gel shaft method, foot print method and the like may be used. When the test substance is a compound of relatively low molecular weight, a BIACORE method, for example, a method described in "Kazuhiro Nagata, Hiroshi Handa collaboration, Seitaibusshitsu Sougosayo no Riarutaimu Kaiseki Jikkenho - BIACORE wo chushin ni - Supringer Verlag Tokyo K.K. "may be used. For example, in the case of a gel shaft method, first, mixture of the polynucleotide of the present invention and a test substance is applied to polyacrylamide gel electrophoresis by a conventional method. When a polyacrylamide gel after electrophoresis is dried followed by detecting the position of the polynucleotide on the gel by, for example, subjecting the gel to autoradiography and the like, whether the polynucleotide is bound to a test substance or not can be checked.

Specifically, for example, the dried gel is exposed to an imaging plate for about 1 hour to about one night, more preferably for 6 to 8 hours, and a picture image is obtained by an imaging analyzer. When a test substance is bound to the polynucleotide, the mobility of the polynucleotide-test substance complex becomes lower than that of a free polynucleotide, causing band shift on the picture image. A test substance in the case of detection of band shift can be selected as the substance which binds to the polynucleotide of the present invention.

Since thus selected binding substance can bind to the polynucleotide of the present invention, the substance can control the transcription controlling ability of the polynucleotide of the present invention. Further, it can control the expression in a cell of a gene under control of the polynucleotide of the present invention. Therefore, this substance or its pharmaceutically acceptable salt may also be utilized in the form of transcription controlling agent containing them as an active ingredient and wherein the active ingredient is formulated in a pharmaceutically acceptable carrier. This substance is useful as a medicine for diseases ascribable to excess expression or under expression of a translation product of the gene.

This substance can also be utilized as a transcription controlling agent in investigating an action in a neuron and an influence on behavior, memory, diseases (particularly, neurological disorder and/or psychiatric diseases) ascribable to abnormality of the expression amount of Gm1 or to abnormality of signal transduction through a promoter of a Gm1 gene and the like, of desired genes, for example, genes of which correlation with neurological disorder and/or psychiatric diseases is presumed, genes of which correlation with behavior or memory is presumed and the like, said genes being linked under control of the polynucleotide of the present invention.

Next, the purification method of the present invention will be illustrated below.

The purification method of the present invention contains a first step of contacting the polynucleotide of the present invention with a sample to form a complex of the polynucleotide with a substance, wherein said substance is contained in the sample and binds to the polynucleotide, and a second step of isolating the binding substance from the formed complex, after the first step.

In contacting the polynucleotide of the present invention with a sample, it is usually preferable to contact the polynucleotide in the form of binding to a carrier with a sample since then the polynucleotide or a complex of the polynucleotide with the binding substance can be recovered easily. The kind of the carrier to which the polynucleotide is bound is not particularly restricted, and for example, commercially available carriers for affinity chromatography, preferably, cyanogen bromide-activated sepharose 4B (manufactured by Amersham Pharmacia Biotech) and the like can be used. A method of binding the polynucleotide directly to a carrier and a method of binding through a spacer are mentioned to bind the polynucleotide to a carrier. In binding, for example, the polynucleotide and cyanogen bromide-activated sepharose 4B are mixed and stirred at about 4°C to about 10°C overnight at 1000 rpm, to fix the polynucleotide on the sepharose. Then, for blocking unreacted cyanogen bromide active groups, the product is, for example, left overnight at about 4°C to about 10°C in a buffer containing a compound carrying an amino group, for example, in a sodium hydrogen carbonate solution containing 1 M glycine. The resulting gel may be contacted with a sample by a conventional batch method, and the gel may be filled in a commercially available chromatograph tube to prepare an affinity column for a substance which binds to the polynucleotide of the present invention and may be contacted with a sample by a conventional column chromatography method.

For example, when contact and isolation are carried out by a column chromatography method, a sample is subjected to the above-mentioned affinity column to form a complex of the polynucleotide of the present invention with the polynucleotide binding substance, then, washing of the carrier and elution of the binding substance are carried out by conventional methods, thus, the binding substance can be isolated. Specifically, first, a sample is loaded on the above-mentioned affinity column, then, the column is washed with a buffer of the same composition as in loading to remove sample components having not formed a complex. Thereafter, gradient elution which increases the concentration of a salt contained in the buffer is performed to elute a substance which has been bound to the polynucleotide of the present invention from the column, thus, the substance which binds to the polynucleotide of the present invention can be isolated. Examples of the above-mentioned salt to be used for elution include sodium chloride, potassium chloride, ammonium sulfate and the like.

The purification method of the present invention can purify a substance selected by the above-mentioned selection method among substances which controls the transcription controlling ability of the polynucleotide of the present invention, or a substance selected by the above-mentioned selection method among substances which binds to the polynucleotide of the present invention.

The transcription controlling agent containing as an active ingredient a substance selected by the searching method of the present invention or the binding substance searching method of the present invention or its pharmaceutically acceptable salt can be administered in its effective amount to a mammal such as human and the like orally or parenterally. For example, in the case of oral administration, the transcription controlling agent can be used in usual form such as tablet, capsule, syrup, suspension and the like. In the case of parenteral administration, the transcription controlling agent can be used in usual liquid form such as solution, emulsion, suspension and the like. As the method of parenterally administering the transcription controlling agent in the above-mentioned form, for example, an injection method, a method of administering in the form of suppository to rectum, and the like are mentioned.

The above-mentioned suitable administration form can be produced by compounding a substance selected by the searching method or its pharmaceutically acceptable salt into an acceptable usual carrier, excipient, binder, stabilizer, diluting agent or the like. In the case of use in injection form, acceptable buffers, dissolution aids, isotonic agents and the like can also be added.

The dosage varies depending on the age, sex, body weight and disease extent of an animal receiving administration, and the kind and administration form of the transcription controlling agent, and the like, and usually, in the case of oral administration, it may be advantageous that the active ingredient amount per day for an adult is about 1 mg to about 2 g, preferably about 5 mg to about 1 g, and in the case of injection, it may be advantageous that the active ingredient amount for an adult is about 0.1 mg to about 500 mg.

The above-mentioned daily dose can be administered in one time or in division in several times.

As the disease to which the transcription controlling agent can be applied, mentioned are diseases (particularly, neurological disorder and/or psychiatric diseases) ascribable to abnormality of signal transduction through a promoter of a G-protein gene, and specific examples thereof include neurological disorder and/or psychiatric diseases such as schizophrenia, manic-depressive psychosis, depression and anxiety. Transcription controlling agent may be used for treatment, improvement and prevention of these diseases.

EXAMPLES

The following examples will illustrate the present invention more in detail, but the present invention is not limited to these examples.

Example 1

(Cloning of polynucleotide comprising mouse Gm1 promoter region)

A polynucleotide comprising a mouse Gm1 promoter region was isolated as described below.

(1) Preparation of probe for phage screening
For performing screening of a phage library, probe DNA comprising a 5' upstream region of mouse Gm1 gene was prepared according to a procedure shown below.
Amplified DNA was obtained by performing PCR, using 1 μg of genomic DNA derived from mouse C57BL/6 as a template, and using 10 μM of forward primer mGmg-1 (5'tgttctcccgacccttcagggatcttcttt; SEQ ID NO: 4), 10 μM of reverse primer mGmg-2 (5'-acctatgagcagcaatggatagagtctat; SEQ ID NO: 5) and TAKARA Taq polymerase (TAKARA LATaq with GC Buffer, manufactured by Takara Shuzo Co., Ltd.).
Regarding PCR conditions, one cycle including incubation at 95°C for 30 seconds, then at 65°C for 30 seconds, and then at 72°C for 2 minutes was repeated 35 times.
The resultant DNA was subjected to agarose gel electrophoresis, then, purified using QIAquick Gel Extraction kit (manufactured by QIAGEN), and recovered. The recovered DNA was used in the following experiment as template DNA for probe.
Next, probe DNA labeled with alkali phosphatase was prepared using 10 μl of the above-mentioned template DNA for probe and Alkphos Direct DNA labeling and detection kit (Amersham Bioscience) according to a protocol appended to the kit.
(2) Screening of genomic library
A mouse Gm1 promoter was isolated using a mouse 129/sv-derived genomic library (STRATAGENE) according to the following procedure.
$1 \times 10^6$ phages were spread on 20 of 15 cm NZY plates, and cultured at 37°C for 8 hours, to form plaques. Nitrocellulose membranes were contacted with the plates containing formed plaques, to transfer the formed plaques onto the nitrocellulose membranes. The nitrocellulose membranes and probe DNA labeled with alkali phosphatase were hybridized using Alkphos Direct DNA labeling and detection kit (Amersham Bioscience) according to a protocol appended to the kit. Detection of hybridization signals was also carried out using Alkphos Direct DNA labeling and detection kit (Amersham Bioscience) according to a protocol appended to the kit.

From a phage on which signal was detected by hybridization, DNA was recovered using Wizard Lambda Preps kit (PROMEGA) according to the appended protocol. Thus, a polynucleotide comprising a mouse Gm1 promoter region was cloned.

Example 2

(Construction of reporter vector containing mouse Gm1 promoter region)

Reporter vectors containing a polynucleotide comprising a mouse Gm1 promoter region, pGL-Gm1 and pGL-Gm1/SacI, were prepared as described below.

The phage DNA comprising a mouse Gm1 promoter region obtained in Example 1 was digested with SacI. The resulting digest was subjected to agarose gel electrophoresis, then, purified using QIAquick Gel Extraction kit (manufactured by QIAGEN) to recover DNA. The recovered DNA was used in the subsequent experiment as insert DNA. pGL3 was digested with SacI, then, treated with alkali phosphatase, to prepare vector DNA. Then, 50 ng of the prepared vector DNA and 10 ng of the above-mentioned insert DNA were ligated using a T4 ligase, to prepare Gm1 promoter reporter vector pGL-Gm1/SacI. In this vector pGL-Gm1/SacI, the nucleotide sequence of SEQ ID NO: 2 (this sequence corresponds to the nucleotide sequence of the nucleotide numbers 603 to 3871 of SEQ ID NO: 1) is inserted at a SacI site of pGL3.

Further, the phage DNA comprising a mouse Gm1 promoter region obtained in Example 1 was digested with NheI. The resulting digest was subjected to agarose gel electrophoresis, then, purified using QIAquick Gel Extraction kit (manufactured by QIAGEN) to recover DNA. The recovered DNA was used in the subsequent experiment as insert DNA. Then, pGL3 was digested with NheI, then, treated with alkali phosphatase, to prepare vector DNA. 50 ng of the prepared vector DNA and 10 ng of the insert DNA were ligated using a T4 ligase, to prepare Gm1 promoter reporter vector pGL-Gm1/NheI. Next, pGL-Gm1/SacI was double-digested with NdeI and XhoI, the resultant digest was subjected to agarose gel electrophoresis, then, 2 kb of DNA was purified using QIAquick Gel Extraction kit (manufactured by QIAGEN) to recover. The recovered DNA was used in the subsequent experiment as insert DNA. pGL-Gml/NheI was double-digested with NdeI and XhoI. 50 ng of the resultant digest (vector DNA) and 10 ng of the insert DNA were ligated using a T4 ligase, to prepare Gm1 promoter reporter vector pGL-Gm1. In this vector pGL-Gm1, the nucleotide sequence of SEQ ID NO: 1 is inserted between a SacI site and a NheI site of pGL3.

Example 3

(Measurement of transcription activity of mouse Gm1 promoter)

The transcription-activating ability of the Gm1 promoter of the present invention obtained in Example 1 was measured as described below.

PC12 cells were inoculated into wells of a 96-well plate at a rate of $5 \times 10^4$ cells for each well, and cultured for about 24 hours. Into the cultured cell, the Gm1 promoter reporter vector pGL-Gm1 (0.2 $\mu$g) or pGL-Gm1/SacI (0.2 $\mu$g) obtained in Example 2 was transfected using a lipofection method, to prepare test cells. Into cells inoculated in the same manner as described above, a reporter vector (pGL3; 0.2 $\mu$g) containing no Gm1 promoter was transfected using a lipofection method, to prepare control cells.

Next, these cells were cultured at 37°C for 24 hours, then, the medium in the wells was removed, before washing with a PBS buffer solution. The washed cells were lysed with cell lysis solution (PicaGene Luciferase kit, manufactured by Toyo Ink), then, a luminescence substrate (PicaGene Luciferase kit, manufactured by Toyo Ink) was added into the wells, and the luminescence level of each well was measured using a luminometer. As control, the luminescence level of the control cell was also measured in the same manner. The results are shown in Fig. 1.

When the luciferase activity of the test cell showed 130% or more, where the luciferase activity of the control cell was normalized as 100%, the test cell was recognized to have a transcription-controlling ability.

Example 4

(Immunostaining using anti-Gm1 antibody in brain tissue)

Distribution of the expression of $\alpha$ subunit Gm1 protein of trimeric G-protein in brain was analyzed by a fluorescent tissue immunostaining method using an anti-Gm1 antibody. That is, a mouse brain section (manufactured by Novagen) fixed using para-formaldehyde was subjected to deparaffinization, then, autoclaved (120°C, 15 minutes) in a 0.01 M citric acid solution.

Next, a brain section was incubated at 4°C for 1 hour in an anti-Gm1 antibody (1/100 dilution) diluted using a blocking reagent appended to TSA kit #12 (Molecular Probe). Then, the brain section was washed twice with a PBS solution, then, incubated at 4°C for 1 hour in a HRP-labeled anti-rabbit IgG antibody appended to TSA kit #12 (Molecular Probe). Then, the brain section was washed twice with a PBS solution, then, fluorescent signals were detected using a tyramide signal amplification kit appended to TSA kit #12 (Molecular Probe) according to a protocol appended to the kit. The fluorescent signals were observed using a fluorescent microscope. The results are shown in Fig. 2. It was clarified that the $\alpha$ subunit Gm1 protein of trimeric G-protein expressed highly in neuron of cerebral cortex, hippocampus and cerebellum.

Example 5

(Immunostaining using anti-Gm1 antibody in brain tissue of psychiatric diseased patient)

The expression of $\alpha$ subunit Gm1 protein of trimeric G-protein in brain of psychiatric diseased human patient was analyzed by a tissue immunostaining method using an anti-Gm1 antibody.

That is, a human brain section slide LandMark Low density Neuropsychiatric Tissue MicroArrays (manufactured by Ambion) fixed using para-formaldehyde was subjected to deparaffinization, then, autoclaved (120°C, 15 minutes) in a 0.01 M citric acid solution.

Next, the slide containing a brain section (hereinafter, abbreviated as "slide") was washed with ultrapure water, then, incubated at room temperature for 10 minutes in methanol containing 3% hydrogen peroxide water.

Next, the incubated slide was washed three times with a TN solution (0.1 M Tris pH 7.5, 0.15 M NaCl), then, incubated at room temperature for 30 minutes in a blocking reagent appended to TSA biotin system NEL 700 (manufactured by Perkin Elmer).

Next, the slide was incubated at 4°C for 1 hour in an anti-Gm1 antibody (1/100 dilution) diluted using a blocking reagent appended to TSA Biotin System NEL700 (manufactured by Perkin Elmer), then, this was washed three times with a TN solution (0.1 M Tris pH 7.5, 0.15 M NaCl).

Then, the slide was incubated at 4°C for 1 hour in a HRP-labeled anti-rabbit IgG antibody (1/200 dilution) diluted using a blocking reagent appended to TSA Biotin System NEL700 (manufactured by Perkin Elmer), then, this was washed three times with a TN solution (0.1 M Tris pH 7.5, 0.15 M NaCl).

Then, the slide was incubated at room temperature for 10 minutes in a biotinated tyramide amplification reagent appended to TSA Biotin System NEL700 (manufactured by Perkin Elmer), then, this was washed three times with a TN solution (0.1 M Tris pH 7.5, 0.15 M NaCl).

Then, the slide was incubated at room temperature for 30 minutes in an anti-HRP-labeled anti-avidin antibody appended to TSA Biotin System NEL700 (manufactured by Perkin Elmer), then, this was washed three times with a TN solution (0.1 M Tris pH 7.5, 0.15 M NaCl).

The slide thus obtained was allowed to develop color using one DAB tablet (manufactured by Sigma) dissolved in 15 ml of ultrapure water, then, observed by a microscope. The results are shown in Fig. 3. It was clarified that the α subunit Gm1 protein of trimeric G-protein expressed lower in the brain of a patient with neurological disorder and/or psychiatric diseases (schizophrenia, manic-depressive psychosis, depression and anxiety) than in healthy subject.

Example 6

(Construction of reporter vector containing mouse Gm1 promoter region)

A reporter vector containing a nucleotide sequence of a mouse Gm1 promoter region, pGL-Gm1/843-3900, is constructed as described below.

For amplifying DNA comprising a nucleotide sequence of the nucleotide numbers 843 to 3871 of SEQ ID NO: 1 to which 29 nucleotides are added at the 3' end, PCR is carried out using 10 ng of the phage DNA comprising a mouse Gm1 promoter region obtained in Example 1, and using 10 μM of forward primer prmGmg-843 (5'-atcatcacaacaggaaagtaataaaa; SEQ ID NO: 6), 10 μM of reverse primer prmGmg-3900 (5'-attgtgcatccttcgaacgtccca; SEQ ID NO: 7) and TAKARA LA Taq polymerase (TAKARA LA Taq with GC Buffer, manufactured by Takara Shuzo Co., Ltd.).

Regarding PCR conditions, one cycle including incubation at 95°C for 30 seconds, then at 60°C for 30 seconds, and then at 72°C for 2 minutes is repeated 35 times. The resultant DNA is subjected to agarose gel electrophoresis, then, purified using QIAquick Gel Extraction kit (manufactured by QIAGEN), and recovered. The recovered DNA is used in the following experiment as insert DNA.

Plasmid pDrive-Gm1/843-3900 is obtained by ligating 10 ng of insert DNA and 50 ng of pDrive vector (manufactured by QIAGEN) using a T4 ligase.

The resultant plasmid pDrive-Gm1/843-3900 was double-digested with MluI and SacI. The resultant digest is subjected to agarose gel electrophoresis, then, DNA is purified using QIAquick Gel Extraction kit (manufactured by QIAGEN) to recover. The recovered DNA is used in the subsequent experiment as insert DNA. pGL-3 is double-digested with MluI and SacI. 50 ng of the resultant digest (vector) and 10 ng of the insert DNA are ligated using a T4 ligase, to prepare Gm1 promoter reporter vector pGL-Gm1/843-3900.

DNA comprising a nucleotide sequence of the nucleotide numbers 1232 to 3871 of SEQ ID NO: 1 to which 29 nucleotides are added at the 3' end is amplified, using 10 μM of forward primer prmGmg-1232 (5'-tggtgccctcttctggtgtgtct; SEQ ID NO: 8) and 10 μM of reverse primer prmGmg-3900 (5'-attgtgcatccttcgaacgtccca; SEQ ID NO: 7), and a reporter vector containing a nucleotide sequence in a mouse Gm1 promoter region, pGL-Gm1/1232-3900, is constructed according to the same procedure as described above.

DNA comprising a nucleotide sequence of the nucleotide numbers 1989 to 3871 of SEQ ID NO: 1 to which 29 nucleotides are added at the 3' end is amplified, using 10 μM of forward primer prmGmg-1989 (5'-atgccatatacttgagtcacagtttgtgaa; SEQ ID NO: 9) and 10 μM of reverse primer prmGmg-3900 (5'-attgtgcatccttcgaacgtccca ; SEQ ID NO: 8), and a reporter vector containing a nucleotide sequence in a mouse Gm1 promoter region, pGL-Gm1/1989-3900, is constructed according to the same procedure as described above.

DNA comprising a nucleotide sequence of the nucleotide numbers 2937 to 3871 of SEQ ID NO: 1 to which 29 nucleotides are added at the 3' end is amplified, using 10 μM of forward primer prmGmg-2937 (5'-ctcccgagccttcagggatcttcttt; SEQ ID NO: 10) and 10 μM of reverse primer prmGmg-3900 (5'-attgtgcatccttcgaacgtccca; SEQ ID NO: 8), and a reporter vector containing a nucleotide sequence in a mouse Gm1 promoter region, pGL-Gml/2937-3900, is constructed according to the same procedure as described above.

DNA comprising the nucleotide sequence of the nucleotide numbers 843 to 3776 of SEQ ID NO: 1 is amplified,

using 10 μM of forward primer prmGmg-843 (5'-atcatcacaacagggaaagtaataaaa; SEQ ID NO: 6) and 10 μM of reverse primer prmGmg-3776 (5'-agactgctccggttaccgtaaatactgcct; SEQ ID NO: 11), and a reporter vector containing a nucleotide sequence in a mouse Gm1 promoter region, pGL-Gm1/843-3776, is constructed according to the same procedure as described above.

DNA comprising the nucleotide sequence of the nucleotide numbers 1232 to 3776 of SEQ ID NO: 1 is amplified, using 10 μM of forward primer prmGmg-1232 (5'-tggtgccctcttctggtgtgtct; SEQ ID NO: 8) and 10 μM of reverse primer prmGmg-3776 (5'-agactgctccggttaccgtaaatactgcct; SEQ ID NO: 11), and a reporter vector containing a nucleotide sequence in a mouse Gm1 promoter region, pGL-Gm1/1232-3776, is constructed according to the same procedure as described above.

DNA comprising the nucleotide sequence of the nucleotide numbers 1989 to 3776 of SEQ ID NO: 1 is amplified, using 10 μM of forward primer prmGmg-1989 (5'-atgccatatacttgagtcacagtttgtgaa; SEQ ID NO: 9) and 10 μM of reverse primer prmGmg-3776 (5'-agactgctccggttaccgtaaatactgcct; SEQ ID NO: 11), and a reporter vector containing a nucleotide sequence in a mouse Gm1 promoter region, pGL-Gm1/1989-3776, is constructed according to the same procedure as described above.

DNA comprising the nucleotide sequence of the nucleotide numbers 2937 to 3776 of SEQ ID NO: 1 is amplified, using 10 μM of forward primer prmGmg-2937 (5'-ctcccgagccttcagggatcttcttt; SEQ ID NO: 10) and 10 μM of reverse primer prmGmg-3776 (5'-agactgctccggttaccgtaaatactgcct; SEQ ID NO: 11), and a reporter vector containing a nucleotide sequence in a mouse Gm1 promoter region, pGL-Gm1/2937-3776, is constructed according to the same procedure as described above.

Example 7

(Determination of transcription-controlling region of mouse Gm1 promoter)

The transcription-activating ability of the Gm1 promoter of the present invention is measured as described below using the reporter vector obtained in Example 6.

PC12 cells were inoculated into wells of a 96-well plate at a rate of $5 \times 10^4$ cells for each well, and cultured for about 24 hours. Into the cultured cell, the Gm1 promoter reporter vector (pGL-Gm1/843-3900, pGL-Gm1/1232-3900, pGL-Gm1/1989-3900, pGL-Gm1/2937-3900, pGL-Gm1/843-3776, pGL-Gm1/1232-3776, pGL-Gm1/1989-3776 or pGL-Gm1/2937-3776; each 0.2 μg) obtained in Example 6 is transfected using a lipofection method, to prepare test cells. Using cells inoculated in the same manner as described above, a reporter vector (pGL3; 0.2 μg) containing no Gm1 promoter is transfected using a lipofection method, to prepare control cells.

Next, these cells are cultured at 37°C for 24 hours, then, the medium in the well is removed, before washing with a PBS buffer solution. The washed cells are lysed with cell lysis solution (PicaGene Luciferase kit, manufactured by Toyo Ink), then, a luminescence substrate (PicaGene Luciferase kit, manufactured by Toyo Ink) is added into the wells, and the luminescence level of each well is measured using a luminometer. As control, the luminescence level of the control cell is also measured in the same manner.

When the luciferase activity of the test cell showes 130% or more, where the luciferase activity of the control cell is normalized as 100%, the test cell is recognized to have a transcription-controlling ability.

Example 8

(Screening using reporter activity as index)

PC12 cells were inoculated into wells of a 96-well plate at a rate of $5 \times 10^4$ cells for each well, and cultured for about 24 hours. Into the cultured cell, the Gm1 promoter reporter vector (pGL-Gm1; 0.2 μg) obtained in Example 2 is transfected using a lipofection method, to prepare test cells.

Next, these cells are cultured for about 24 hours, then, the medium in the well is removed. Into the well, 0.10 ml of a fresh medium is newly added, further, 0.1 nM to 10 nM of a test substance is added, and this is cultured at 37°C for 24 hours.

Next, the medium in the well is removed, before washing with a PBS buffer solution. The washed cells are lysed with cell lysis solution (PicaGene Luciferase kit, manufactured by Toyo Ink), then, a luminescence substrate (PicaGene Luciferase kit, manufactured by Toyo Ink) is added into the wells, and the luminescence level of each well is measured using a luminometer. As control, the luminescence level of cells of no contact with the test substance is also measured in the same manner.

When the luciferase activity percentage in the case of contact with a test substance is 50% or less, or 150% or more, where the luciferase activity in the case of no contact with the test substance is normalized as 100%, the substance is selected as a signal transduction controlling substance.

Example 9

(Screening using reporter activity as index)

The transcription activating ability of the Gm1 promoter of the present invention obtained in Example 2 is measured as described below.

PC12 cells are inoculated into wells of a 96-well plate at a rate of $5\times10^4$ cells for each well, and cultured for about 24 hours. Into the cultured cell, the Gm1 promoter reporter vector (pGL-Gm1 or pGL-Gm1/SacI each 0.2 μg) obtained in Example 2 is transfected using a lipofection method, to prepare test cells. A reporter vector (pGL3; 0.2 μg) containing no Gm1 promoter is transfected using a lipofection method, to prepare control cells.

Next, these cells are cultured for about 24 hours, then, the medium in the well is removed. Into the well, 0.10 ml of a fresh medium is newly added, then, further, 0.1 nM to 10 nM of a test substance is added, and this is cultured at 37°C for 24 hours.

Next, the medium in the well is removed, before washing with a PBS buffer solution. The washed cells are lysed with cell lysis solution (PicaGene Luciferase kit, manufactured by Toyo Ink), then, a luminescence substrate (PicaGene Luciferase kit, manufactured by Toyo Ink) is added into the wells, and the luminescence level of each well is measured using a luminometer. As control, the luminescence level of a cell not contacted with a test substance is also measured in the same manner.

When ratios of the luciferase activity in test cells in the case of contact with a test substance to in the case of no contact with the test substance is 1.3-fold or more, or 0.7-fold or less, as compared with the control cell, the substance is selected as a signal transduction substance.

Example 10

(Selection of substance which binds to polynucleotide of the present invention)

Five (5) μg of the plasmid pGL-Gm1 of the present invention described in Example 3 is digested at 37°C for 1 hour in 20 μL of a reaction solution using each 10 U of XhoI and MluI. The restriction enzyme-digested reaction solution is subjected to agarose gel electrophoresis, and insert DNA is purified using QIAquick Gel Extraction kit. One (1) μg of the resultant DNA, 5 μL of [$\alpha$-$^{32}$P]dCTP (manufactured by Amersham Pharmacia Biotech), no-labeled nucleotides (dATP, dTTP, dGTP)(Takara Shuzo Co., Ltd.) each 1 μL, 2 μL of 10×Klenow Buffer (Takara Shuzo Co., Ltd.) and 1 μL of Klenow enzyme (Takara Shuzo Co., Ltd.) are added and distilled water is added to give a total amount of 20 μL, and the mixture is incubated at 37°C for 1 hour, to obtain labeled DNA. Next, for separating unreacted [$\alpha$-$^{32}$P]dCTP and the labeled DNA, the reaction solution is subjected to ProbeQuant G-50 Micro Columns (Amersham Pharmacia Biotech) equilibrated with 10 mM Tris acetate and 1 mM EDTA (hereinafter, referred to as TE solution), and centrifuged (room temperature, 1200 rpm, 1 minute), to elute the labeled DNA. The radioactivity of the resulting eluate is measured by scintillator, and diluted with the TE solution to give $10^4$ cpm/μL to obtain a labeled DNA solution. 5 μL of 5×binding buffer (containing 50 mM Hepes-potassium hydroxide (pH 7.8), 250 mM potassium chloride, 5 mM EDTA (pH 8.0), 25 mM magnesium chloride, 50% (W/V) glycerol, 25 mM dithiothreitol, 3.5 mM PMSF, 10 μg/mL Aprotinin, 10 μg/mL Pepstatin, 10 μg/mL Leupeptin and 5 mM sodium orthovanadate), 1.5 μL of 2 μg/pL polydIdC, 5 μL of 10 μM test substance and 2 μL of the labeled DNA solution are mixed and distilled water is added to give a total amount of 25 μL. The solution is incubated at room temperature for 30 minutes, then, subjected to polyacrylamide gel electrophoresis. After electrophoresis, the gel is peeled from a gel plate, and dried on a Whatman 3MM filter paper at 80°C for 1 hour using a vacuum pump, then, exposed to an imaging plate for 2 hours, then, a picture image is obtained using BAS2000. When, as a result of binding of a test substance to the labeled DNA to form a labeled DNA-test substance complex, the mobility of the complex on the gel becomes smaller than free labeled DNA and band shift on the picture image is detected, the test substance is selected as the substance which binds to the polynucleotide of the present invention.

Example 11

(Purification of substance which binds to polynucleotide of the present invention by purification method of the present invention)

(1) Preparation of affinity column for purification of substance which binds to polynucleotide of the present invention
200 μg of the plasmid pGL-Gm1 of the present invention is digested at 37°C for 1 hour in 200 μL of a reaction solution using each 50 U of XhoI and MluI. The restriction enzyme-digested reaction solution is subjected to agarose gel electrophoresis, and purified using QIAquick Gel Extraction kit to obtain the DNA. 44 μg of the DNA is added to 2 mL of cyanogen bromide-activated sepharose 4B and stirred at 4°C overnight at 1000 rpm, to fix the DNA on the sepharose. Then, for blocking unreacted cyanogen bromide active groups, 20 mL of a sodium hydrogen carbonate solution (pH 9.5) containing 1 M glycine is added and the mixture is left overnight at 4°C. The gel thus obtained is filled in 10×300 mm chromatograph tube (manufactured by Iwaki Glass), to prepare an affinity column for a substance which binds to the polynucleotide of the present invention.

(2) Preparation of sample

Human nerve-derived cultured cells IMR-32 are inoculated on 40 pieces of 15 cm plates (manufactured by Falcon) at a rate of $1 \times 10^7$ cells in each plate. Using a D-MEM medium (high glucose) containing 10% (W/V) FBS added, culturing is performed for two nights in the presence of 5% (V/V) carbon dioxide at 37°C. After two nights, the medium is removed, then, the vessel wall of the plate is washed once with 15 mL of phosphate buffer, then, 2 mL of a trypsin-EDTA solution (containing 0.05% (W/V) trypsin and 0.53 mM of EDTA; manufactured by Gibco) is added to the plate so as to immerse cells, and allowed to stand at 37°C for 5 minutes. To this is added a FBS-containing medium in an amount of about 10-fold of the trypsin-EDTA solution, to obtain a cell suspension. The resultant cell suspension is centrifuged (room temperature, 1300 rpm, 5 minutes) to remove the supernatant. The cell precipitate is suspended in 15 mL of phosphate buffer, and again centrifuged at room temperature at 1300 rpm for 5 minutes to remove the supernatant. Thereafter, the cell precipitate is suspended in ice-cold 10 mM Hepes-potassium hydroxide (pH 7.8), 10 mM potassium chloride and 10 mL of 0.1 mM EDTA (pH 8.0) solution (hereinafter; referred to as buffer A). After cooling for 10 minutes on ice, the cell suspension is centrifuged at 4°C at 1300 rpm for 5 minutes. The supernatant is removed, then, the cell precipitate is re-suspended in 30 ml of buffer A, and cells are disrupted completely while cooling on ice using Downs Homogenizer Pessel B (manufactured by Wheaton). The resulting cell homogenate is centrifuged (4°C, 1300 rpm, 5 minutes) to remove the supernatant. The precipitate is suspended in 50 mM Hepes-KOH (pH 7.8), 420 mM potassium chloride, 0.1 mM EDTA (pH 8.0), 5 mM magnesium chloride and 2 mL of 2% (W/V) glycerol solution (hereinafter, referred to as buffer B). After rotating gently at 4°C for 30 minutes by a rotator, the suspension is centrifuged (4°C, 24000 g, 30 minutes). The supernatant is recovered and diluted 5-fold with distilled water to give a diluted solution as a sample of purification.

(3) Step of isolation

The sample prepared in the above-mentioned step (2) is loaded on the affinity column described in the above-mentioned step (1). Further, 10 mL of 5-fold diluted buffer B is loaded, to remove sample components not formed a complex. Thereafter, gradient elution to increase the potassium chloride concentration up to 1 M is performed to cause elution of the substance which binds to the polynucleotide of the present invention, obtaining a fraction containing the substance which binds to the polynucleotide of the present invention.

## INDUSTRIAL APPLICABILITY

The present invention can provide polynucleotides comprising a nucleotide sequence of a promoter region of α subunit Gm1 of trimeric G-protein. The polynucleotides are useful for research and development of a method of searching signal transduction controlling substance through a promoter of α subunit Gm1 of trimeric G-protein, and medicines for neurological disorder and/or psychiatric diseases containing, as an active ingredient, a compound having a signal transduction controlling ability through a promoter of α subunit Gm1 of trimeric G-protein obtained by the above-mentioned searching method or its pharmaceutically acceptable salt.

Free Text in Sequence Listing

SEQ ID NO: 4

Designed oligonucleotide primer for PCR

SEQ ID NO: 5

Designed oligonucleotide primer for PCR

SEQ ID NO: 6

Designed oligonucleotide primer for PCR

SEQ ID NO: 7

Designed oligonucleotide primer for PCR

SEQ ID NO: 8

Designed oligonucleotide primer for PCR

SEQ ID NO: 9

Designed oligonucleotide primer for PCR

SEQ ID NO: 10

Designed oligonucleotide primer for PCR

SEQ ID NO: 11

Designed oligonucleotide primer for PCR

## Claims

1. A polynucleotide comprising a nucleotide sequence of a promoter region of a gene encoding α subunit Gm1 of trimeric G-protein.

**2.** The polynucleotide according to claim 1, wherein the nucleotide sequence of a promoter region is any of the following nucleotide sequences (1) to (4):

(1) the nucleotide sequence of SEQ ID NO: 1,
(2) the nucleotide sequence of the nucleotide numbers 603 to 3871 in the nucleotide sequence of SEQ ID NO: 1,
(3) a nucleotide sequence of (1) or (2) with deletion, substitution or addition of one or more nucleotides, said nucleotide sequence having an ability of controlling the transcription of a gene encoding α subunit Gm1 of trimeric G-protein, and
(4) a nucleotide sequence having an ability of controlling the transcription of a gene encoding α subunit Gm1 of trimeric G-protein, and being complementary to a nucleotide sequence of a polynucleotide, wherein said polynucleotide hybridizes under a stringent condition to a polynucleotide comprising the nucleotide sequence of (1) or (2).

**3.** A plasmid comprising the polynucleotide of claim 1 or 2.

**4.** A plasmid comprising the polynucleotide of claim 1 or 2, wherein at the downstream (3' side) of said polynucleotide, said plasmid contains a polynucleotide of which transcription is controlled by said polynucleotide.

**5.** A plasmid comprising the polynucleotide of claim 1 or 2, wherein at the downstream (3' side) of said polynucleotide, said plasmid contains a reporter gene of which transcription is controlled by said polynucleotide.

**6.** A transformed cell in which the polynucleotide of claim 1 or 2 is introduced.

**7.** A transformed cell in which the plasmid of claim 3 or 4 is introduced.

**8.** A transformed cell in which the plasmid of claim 5 is introduced.

**9.** A method for searching a signal transduction controlling substance through a promoter of a gene encoding α subunit Gm1 of trimeric G-protein, comprising

(1) a first step of contacting the transformed cell of claim 8 with a test substance,
(2) a second step of monitoring the expression amount of a reporter gene or an index value correlated therewith, after the first step,
(3) a third step of evaluating an ability of the above-mentioned substance to control signal transduction through a promoter of a gene encoding α subunit Gm1 of trimeric G-protein, based on a change in the expression amount or index value correlated therewith monitored in the second step, and
(4) a fourth step of selecting a substance having an ability to control signal transduction through a promoter of a gene encoding α subunit Gm1 of trimeric G-protein, based on the signal transduction controlling ability evaluated in the third step.

**10.** A method for evaluating an ability of a substance to control signal transduction through a promoter of a gene encoding α subunit Gm1 of trimeric G-protein, comprising

(1) a first step of contacting the transformed cell of claim 8 with a test substance,
(2) a second step of monitoring the expression amount of a reporter gene or an index value correlated therewith, after the first step, and
(3) a third step of evaluating an ability of the above-mentioned substance to control signal transduction through a promoter of a gene encoding α subunit Gm1 of trimeric G-protein, based on a change in the expression amount or index value correlated therewith monitored in the second step.

**11.** A method for searching a substance which binds to the polynucleotide of claim 1, comprising

(1) a first step of contacting the polynucleotide of claim 1 with a test substance,
(2) a second step of checking the presence or absence of formation of a complex of the polynucleotide with the test substance, after the first step, and
(3) a third step of selecting a substance which binds to the polynucleotide, based on the analysis result, obtained in the second step, of the presence or absence of formation of a complex.

**12.** A method for purifying a substance which binds to the polynucleotide of claim 1, comprising

(1) a first step of contacting the polynucleotide of claim 1 with a sample to form a complex of the polynucleotide with a substance, wherein said substance is contained in the sample and binds to the polynucleotide, and
(2) a second step of isolating the substance which binds to the polynucleotide, from a formed complex, after the first step.

**13.** A kit for screening a signal transduction controlling substance through a promoter of a gene encoding α subunit Gm1 of trimeric G-protein, comprising the transformed cell of claim 8 and a reagent for measuring the expression amount of a reporter gene or an index value correlated therewith.

**14.** A medicine for neurological disorder and/or psychiatric diseases comprising as an active ingredient a compound having an ability to control signal transduction through a promoter of a gene encoding α subunit Gm1 of trimeric G-protein, obtained by the searching method of claim 9 or 11, or a pharmaceutically acceptable salt thereof, wherein the active ingredient is formulated in a pharmaceutically acceptable carrier.

Fig.1

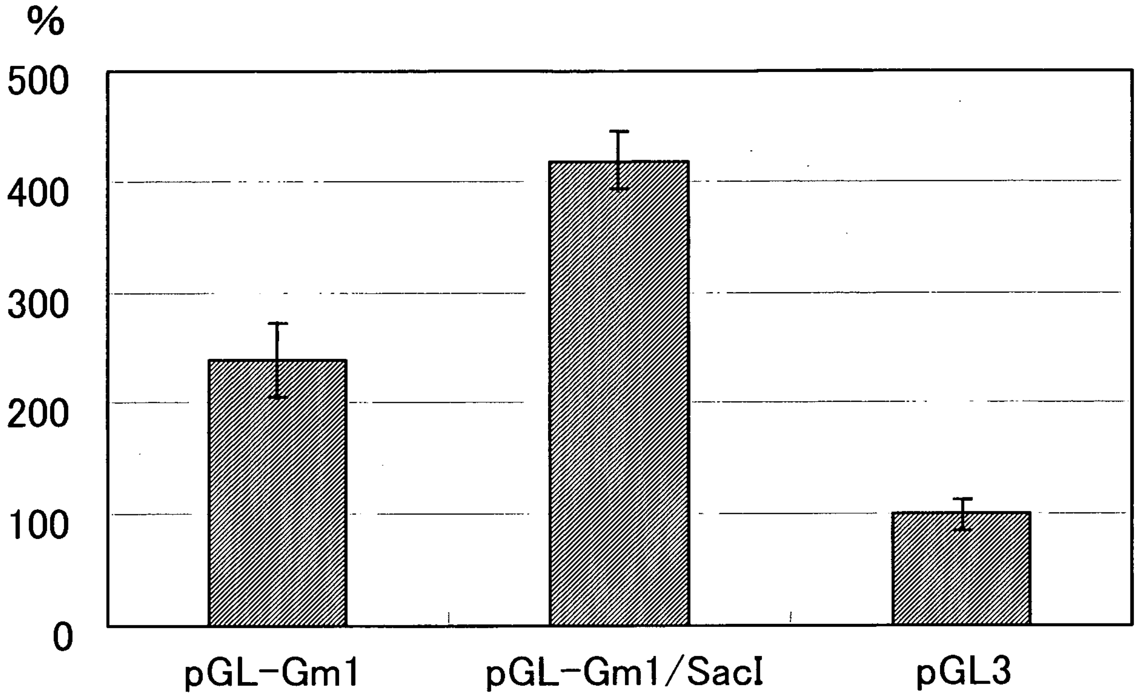

Fig.2

cerebral cortex

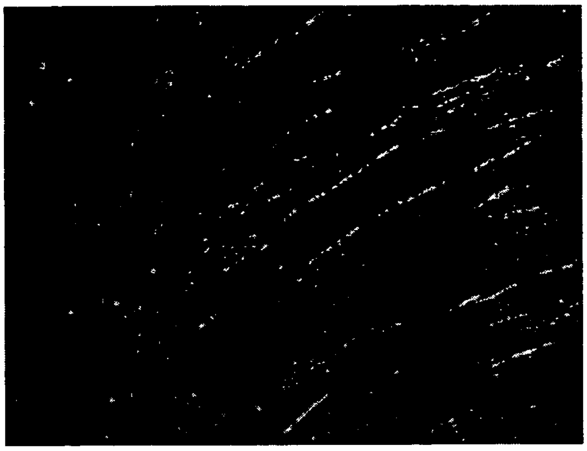

hippocampus

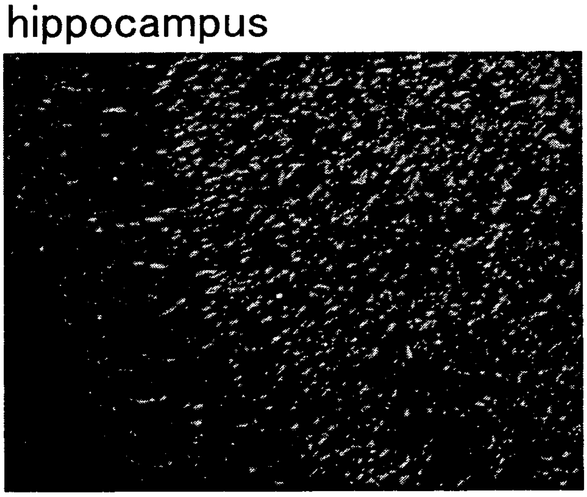

cerebellum

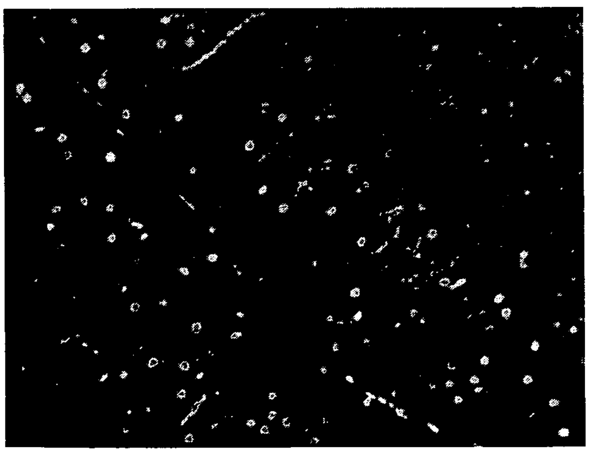

Fig.3

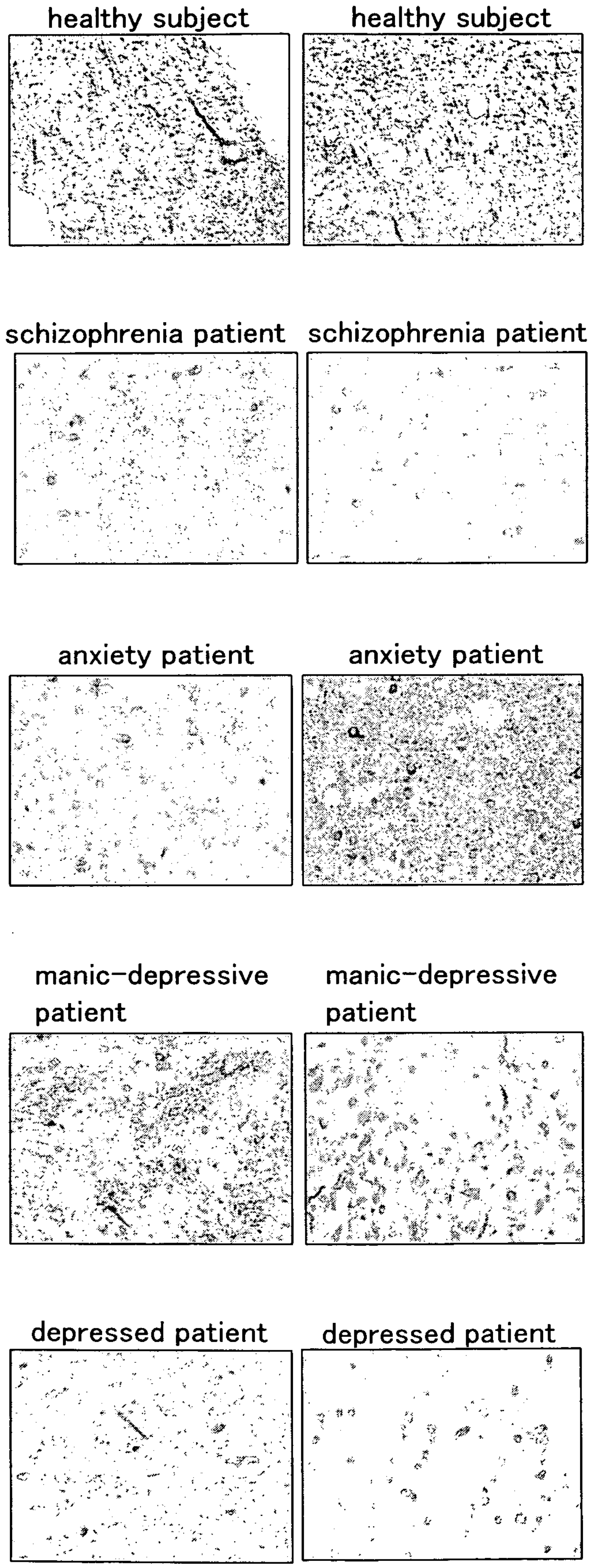
healthy subject
healthy subject
schizophrenia patient
schizophrenia patient
anxiety patient
anxiety patient
manic-depressive patient
manic-depressive patient
depressed patient
depressed patient

**INTERNATIONAL SEARCH REPORT**

International application No.
PCT/JP2005/005077

A. CLASSIFICATION OF SUBJECT MATTER
Int.Cl[7] C12N15/11, A61K45/00, A61P25/18, 25/22, 25/24, C12N1/15, 1/19, 1/21, 5/10, C12Q1/02, 1/68, G01N33/15, 33/50

According to International Patent Classification (IPC) or to both national classification and IPC

B. FIELDS SEARCHED

Minimum documentation searched (classification system followed by classification symbols)
Int.Cl[7] C12N15/11, A61K45/00, A61P25/18, 25/22, 25/24, C12N1/15, 1/19, 1/21, 5/10, C12Q1/02, 1/68, G01N33/15, 33/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
GenBank/EMBL/DDBJ/GeneSeq, SwissProt/PIR/Geneseq, CAplus/BIOSIS/MEDLINE/WPIDS(STN), JSTPlus(STN)

C. DOCUMENTS CONSIDERED TO BE RELEVANT

| Category* | Citation of document, with indication, where appropriate, of the relevant passages | Relevant to claim No. |
|---|---|---|
| X | EP 1382613 A1 (Sumitomo Chemical Co., Ltd.), 21 January, 2004 (21.01.04), & JP 2004-350672 A & CA 2432968 A | 1-13 |
| A | JP 2002-034575 A (Shiseido Co., Ltd.), 05 February, 2002 (05.02.02), & WO 2002/010376 A1 & EP 1306432 A1 | 1-13 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 May, 2005 (26.05.05) | 14 June, 2005 (14.06.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No.
PCT/JP2005/005077

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 14
because they relate to parts of the international application that do not comply with the prescribed requirements to such an extent that no meaningful international search can be carried out, specifically:
With respect to "compounds capable of regulation of signal transmission by means of promoter of gene coding for trimer G protein α-subunit Gm1, obtained by the screening method of claim 9 or 11" claimed in claim 14, what particular (continued to extra sheet)

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest.
☐ No protest accompanied the payment of additional search fees.

Form PCT/ISA/210 (continuation of first sheet (2)) (January 2004)

INTERNATIONAL SEARCH REPORT

International application No.
PCT/JP2005/005077

Continuation of Box No.II-2 of continuation of first sheet(2)

compounds are involved or not involved therein is utterly ambiguous. Thus, claim 14 is extremely unclear, and consequently no meaningful opinion as to the novelty, inventive step and industrial applicability of the invention claimed in claim 14 cannot be presented.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *European Journal of Endocrinology,* 2001, vol. 145, 545-559
- Molecular Cloning: A Laboratory Manual. Cold Spring Harbor Laboratory Press, 1989
- **D.M. GLOVER.** DNA Cloning. IRL, 1985
- **TAKAAKI TAMURA.** *Shin Tensha Seigyo no Mechanism,* 2000, 33-40
- *Datsu Isotope Jikken Protocol,* 1998
- **A. GREENER ; M. CALLAHAN.** *Strategies,* 1994, vol. 7, 32-34
- **W. KRAMER et al.** *Nucleic Acids Research,* 1984, vol. 12, 9441
- **W. KRAMER ; H. J. FRITS.** *Methods in Enzymology,* 1987, vol. 154, 350
- **T. A. KUNKEL.** *Proc. Of Natl. Acad. Sci. U.S.A.,* 1985, vol. 82, 488
- **T. A. KUNKEL et al.** *Methods in Enzymology,* 1987, vol. 154, 367
- **S. HENIKOFF et al.** *Gene,* 1984
- **C. YANISCH-PERRON et al.** *Gene,* 1985, vol. 33, 103
- Current Protocols In Molecular Biology. John Wiley & Sons, Inc, 1987
- **F. SANGER ; S. NICKLEN ; A.R. COULSON.** *Proceedings of National Academy of Science U.S.A.,* 1977, vol. 74, 5463-5467
- **BOEHRINGER.** Random Labelling Kit. Takara Shuzo Co., Ltd,
- **J. SAMBROOK ; E.F. FRISCH ; T. MANIATIS.** Molecular Cloning. Cold Spring Harbor Laboratory Press, 1989
- Cloning and Sequence: Plant Biotechnology Experiment Manual. Noson Bunka sha, 1989
- **D.M. GLOVER.** DNA cloning, a practical approach. IRL PRESS, 1985
- **GRIMM, S. et al.** *Proc. Natl. Acad. Sci. USA,* vol. 93, 10923-10927
- **TING, A.T. et al.** *EMBO J.,* vol. 15, 6189-6196
- **HAWKINS, C.K. et al.** *Proc. Natl. Acad. Sci. USA,* vol. 93, 13786-13790

SEQUENCE LISTING

SEQUENCE LISTING

<110> SUMITOMO CHEMICAL COMPANY, LIMITED

<120> Gm1 promoter and use thereof

<130> S11530W001

<150> JP 2004-072244

<151> 2004-03-15

<160> 11

<210> 1

<211> 3871

<212> DNA

<213> Mouse

<400> 1

gctagcccca atatatatat gcagcacatg cactcctgat acctgcagaa gccagaagag 60

ggaattggat cccctgtaat tggagttaca aatggttgtg agtggcaatg tggggacggg 120

gaacccaggc tgcatcatga gcagcaagtg ctcttaactg ctgagccatc tcttcagccc 180

taaaaataaa atgtttattt tacatgtatg aatgctctgt cttctatgca catcagaaag 240

ggaaccagat ctcatacagg atgggttgta agccaccatg tggtttctgg gaatggaact 300

caggacctct ggaagaacac ccagtgcttt taaccactga gccatctctt taggccccat 360

aaagtaattt tgtaacaaag aaaatgaatg cttaatgtca gctgactgtt aaagtgtgct 420

gtcaaccctg gagtctggtt gatatagcag gtgtcaactc tttagttac ttttctattg 480

ctgtgacaaa atgccatgac caacacaact tacagaagaa agagtttaat tgacttacag 540
tttcaacagg ttagagtcca taatggtgga gcaaaggcat gggtggcagc aggaacagct 600
gagagctcac atctcaaacc acaagcagga agtaaaacta gcctgaagac tgtgttaatc 660
ttttggaact tcattggcag gaagccgggg cacaccaaag cctcctgtca tccccagggc 720
gcactctctg ccttcggcct gtggatggag atgtaagctc tcagctgcct gctttcgcca 780
tagacttcag ccctcaggaa atgtaagcct actttttctc gtttataact catggtgttt 840
ttatcatcac aacaggaaag taataaaagt cgttttatag ttacaaatta aattagctgc 900
attcaaattc tttgtaactg ataatacata tttaagggca tgatgtaatg tgtaatgtat 960
attttacaca gtacataggt taaataaagc atgggcatga tctcatagtg acttttatgt 1020
tgaaggcact acttgtgctc cttcaagact cttgtgacat aagatagagt atggtcgtta 1080
ctccaaagaa accaaaacac taaaattaga aactaccata tcagggctag agagatggct 1140
cagcggttaa gagcactgac tgctcttccg aaggtcctga gttcaaatac cagcaaccac 1200
atggtggctc acaaccatct gtaatgggat ctggtgccct cttctggtgt gtctacaacc 1260
atctgtaatg ggatctggtg ccctcttctg gtgtgtctga agacagctag agtgtactta 1320
gctataataa aaaataaatc tttgggccag agcaaccaga ggtcctgtat tcaattccca 1380
gcaaccacat gatggctcac aacctgtaca gctacagtgt gctcacatac ataatataaa 1440
taaataaatc tagagaaaaa aaagagagag aaagaaacta ccatactttg gtcgatgaga 1500
aggcacaatt agaaaaggcc tgaccagaat gaccttggtg gaagaagggg cccagcttca 1560
aaaattgtgc tctgaactgg gcagtggtag cacatgcctt taatcccaga ggcaggcaga 1620
tttctgagtt cacggccagc ttggtctaca gagtgagttc cagaacagcc aggactatac 1680
agaaaaaccc tgtctcaaaa agctaaaata aataaataaa aataaaaatg tgctctgatc 1740
tctacatgca tgccatggca aggaggcacg tgcatgcata tacatacata tttacataaa 1800
acatttttaa atgtattaga gctaccatat gacctagcca tctattacca ggtatatatc 1860
tagtaggcta gcagaaagcc taatgccagg agtacattac ttcttttgga cttgttggtc 1920
cctgaagtcc caaaagcacc cccaacttta aaagccagta ttggtgctcc tggctgcccc 1980
tcctgaagat gccatatact tgagtcacag tttgtgaaga aattgggctg gccctactga 2040
cagcttcatc tctattggct agctttctta ggaaggtgct aagcatgcta caggagggct 2100
gaggaagtta tatctaggtg tgtgtgtgtg agagagagag agagagagac agacagacag 2160

acagatagac agacagacag acagacagac acagacagac aggagagtag ggggtgggga 2220
ggggaggggg agagggagag agaccatgaa ttcatgcagg gaggaaagag aagagggaaa 2280
tgatataatc acccaatttt tttaaaagta ctcccctctc cctctcttcc cataaaagaa 2340
gtcttcaccc tttaccctct agccttcttc tacgtgcttt tttgtttgtt tgtttgtttg 2400
ttttggagag aggtgtgttt tggttttttt gaggcagagt ttctctgtgt agccctggct 2460
gtcactctgt agaccaggct ggcctcgaac tttgcctccc cagtgctggg attaaaagcg 2520
tgtgccacca cgactccagg ccttagaagc cagagacttg tggctccagg tgtgctccct 2580
gcccctccag ggtccaacca caagcagcct ggcactctgc atcctgtgac actctctgcc 2640
cttgagtgac acaacaacaa caggcagcag cggttagtgt cagctagtat tagggccctg 2700
gagaacttcc cattgagact ctccactcct atcttacagt gaccatgaaa ttatagctct 2760
gaagcagacc tgaagtatct accctcagtc ctgaggggcg ggcagctcca gtgaccccag 2820
ctcctctatt tttctttttt ttcacttttc atagccttcc tccttccaac tctggcttgt 2880
cacctctgcc ttccgacact gcccttcccc actgggcctc aggagctgct ggtggttgct 2940
ctcccgagcc ttcagggatc ttctttgaca tagcggattg ttcccactat tgtttctagc 3000
taagctggat gtatccatga taaagatcac acgcaggcaa ggaaagacca ttcggggaat 3060
ccttttttatt agacctaatg tcgcaatacc atggacacaa cgtgaaaagt agccgaaccc 3120
ccaatttata tagcccggag aaaggcatgg taaggatgca tcatgtaagt gaagaattgt 3180
atttgccccg atcccagcac agcctccagt tccacggccc tggcctctta ctgcttcccc 3240
tcctgctgta aatgagaaga gcttccaggt catctaatag ccaccaaatc ctatcttgct 3300
gaagatactg tcttccaaag ctggcaaggg atgtctgcag tgatggtcac ggctggaatc 3360
aaggccttct ggatccggag tctttgcttc agttgccgtt atccattcag ctggtgtgtg 3420
tcaccgggct tttaagtgta cagagcagga tgctgtttaa tatcctccca gctccaagct 3480
gccaagctta agggaacagt ctgtcggata gactctatcc attgctgctc ataggtctca 3540
ccaaccctct cttgggagtt ttgctcactc atagaaacta acattttcaa cagtgtttaa 3600
caatgctcca tcctgcccca gcaccgtagg tcgcttagtc tctggctcag ccctagctag 3660
tggtaaccta accatccctg caacaaggca aggagttctg cccggcactt atgataggca 3720
gccagggtac caatacttgc cacaggaggc agtatttacg gtaaccggag cagtctgcgc 3780
ggcggtttta cggtaagggg gggggggggg gcgggctggc caaggccctt ggtcagctcc 3840

gcctgcttgg ggcgctctca ggtgcgagct c 3871

<210> 2
<211> 3279
<212> DNA
<213> Mouse

<400> 2
gagctcacat ctcaaaccac aagcaggaag taaaactagc ctgaagactg tgttaatctt 60
ttggaacttc attggcagga agccggggca caccaaagcc tcctgtcatc cccagggcgc 120
actctctgcc ttcggcctgt ggatggagat gtaagctctc agctgcctgc tttcgccata 180
gacttcagcc ctcaggaaat gtaagcctac tttttctcgt ttataactca tggtgttttt 240
atcatcacaa caggaaagta ataaaagtcg ttttatagtt acaaattaaa ttagctgcat 300
tcaaattctt tgtaactgat aatacatatt taagggcatg atgtaatgtg taatgtatat 360
tttacacagt acataggtta aataaagcat gggcatgatc tcatagtgac ttttatgttg 420
aaggcactac ttgtgctcct tcaagactct tgtgacataa gatagagtat ggtcgttact 480
ccaaagaaac caaaacacta aaattagaaa ctaccatatc agggctagag agatggctca 540
gcggttaaga gcactgactg ctcttccgaa ggtcctgagt tcaaatacca gcaaccacat 600
ggtggctcac aaccatctgt aatgggatct ggtgccctct tctggtgtgt ctacaaccat 660
ctgtaatggg atctggtgcc ctcttctggt gtgtctgaag acagctagag tgtacttagc 720
tataataaaa aataaatctt tgggccagag caaccagagg tcctgtattc aattcccagc 780
aaccacatga tggctcacaa cctgtacagc tacagtgtgc tcacatacat aatataaata 840
aataaatcta gagaaaaaaa agagagagaa agaaactacc atactttggt cgatgagaag 900
gcacaattag aaaaggcctg accagaatga ccttggtgga agaaggggcc cagcttcaaa 960
aattgtgctc tgaactgggc agtggtagca catgccttta atcccagagg caggcagatt 1020
tctgagttca cggccagctt ggtctacaga gtgagttcca gaacagccag gactatacag 1080
aaaaaccctg tctcaaaaag ctaaaataaa taaataaaaa taaaaatgtg ctctgatctc 1140
tacatgcatg ccatggcaag gaggcacgtg catgcatata catacatatt tacataaaac 1200

attttaaat gtattagagc taccatatga cctagccatc tattaccagg tatatatcta 1260
gtaggctagc agaaagccta atgccaggag tacattactt cttttggact tgttggtccc 1320
tgaagtccca aaagcacccc caactttaaa agccagtatt ggtgctcctg gctgcccctc 1380
ctgaagatgc catatacttg agtcacagtt tgtgaagaaa ttgggctggc cctactgaca 1440
gcttcatctc tattggctag ctttcttagg aaggtgctaa gcatgctaca ggagggctga 1500
ggaagttata tctaggtgtg tgtgtgtgag agagagagag agagagacag acagacagac 1560
agatagacag acagacagac agacagacac agacagacag gagagtaggg ggtggggagg 1620
ggagggggag agggagagag accatgaatt catgcaggga ggaaagagaa gagggaaatg 1680
atataatcac ccaatttttt taaaagtact cccctctccc tctcttccca taaaagaagt 1740
cttcaccctt taccctctag ccttcttcta cgtgcttttt tgtttgtttg tttgtttgtt 1800
ttggagagag gtgtgttttg gtttttttga ggcagagttt ctctgtgtag ccctggctgt 1860
cactctgtag accaggctgg cctcgaactt tgcctcccca gtgctgggat taaaagcgtg 1920
tgccaccacg actccaggcc ttagaagcca gagacttgtg gctccaggtg tgctccctgc 1980
ccctccaggg tccaaccaca agcagcctgg cactctgcat cctgtgacac tctctgccct 2040
tgagtgacac aacaacaaca ggcagcagcg gttagtgtca gctagtatta gggccctgga 2100
gaacttccca ttgagactct ccactcctat cttacagtga ccatgaaatt atagctctga 2160
agcagacctg aagtatctac cctcagtcct gaggggcggg cagctccagt gaccccagct 2220
cctctatttt ttcttttttt cacttttcat agccttcctc cttccaactc tggcttgtca 2280
cctctgcctt ccgacactgc ccttccccac tgggcctcag gagctgctgg tggttgctct 2340
cccgagcctt cagggatctt ctttgacata gcggattgtt cccactattg tttctagcta 2400
agctggatgt atccatgata aagatcacac gcaggcaagg aaagaccatt cggggaatcc 2460
tttttattag acctaatgtc gcaataccat ggacacaacg tgaaaagtag ccgaaccccc 2520
aatttatata gcccggagaa aggcatggta aggatgcatc atgtaagtga agaattgtat 2580
ttgccccgat cccagcacag cctccagttc cacggccctg gcctcttact gcttcccctc 2640
ctgctgtaaa tgagaagagc ttccaggtca tctaatagcc accaaatcct atcttgctga 2700
agatactgtc ttccaaagct ggcaagggat gtctgcagtg atggtcacgg ctggaatcaa 2760
ggccttctgg atccggagtc tttgcttcag ttgccgttat ccattcagct ggtgtgtgtc 2820
accgggcttt taagtgtaca gagcaggatg ctgtttaata tcctcccagc tccaagctgc 2880

caagcttaag ggaacagtct gtcggataga ctctatccat tgctgctcat aggtctcacc 2940
aaccctctct tgggagtttt gctcactcat agaaactaac attttcaaca gtgtttaaca 3000
atgctccatc ctgccccagc accgtaggtc gcttagtctc tggctcagcc ctagctagtg 3060
gtaacctaac catccctgca acaaggcaag gagttctgcc cggcacttat gataggcagc 3120
cagggtacca atacttgcca caggaggcag tatttacggt aaccggagca gtctgcgcgg 3180
cggttttacg gtaagggggg gggggggggc gggctggcca aggcccttgg tcagctccgc 3240
ctgcttgggg cgctctcagg tgcgagctc 3279

<210> 3
<211> 360
<212> DNA
<213> Mouse

<400> 3
atgggcctat gctacagcct gcggccgctg ctcttcggga gcccagagga caccccgtgt 60
gcggcctcgg aaccctgcgc agaggatgct cagcccagcg ccgccccggc ccctgcctcg 120
atcccagccc cggctcccgt agggaccctg ctccggcgtg gcggcggccg gatcgtcgcg 180
aacgcgcggc cgccaggcga gctgcagagc cgccggcgac aggagcagct acgagccgag 240
gagcgcgagg cggctaaaga ggcgaggaaa gtcagccggg gcatcgaccg catgctgcgc 300
gagcagaagc gggacctgca gcagacgcac cggctcctgc tgctgggggc tggtgagtcc 360

<210> 4
<211> 30
<212> DNA
<213> Artificial Sequence

<220>

<223> Designed oligonucleotide primer for PCR

<400> 4
tgttctcccg acccttcagg gatcttcttt 30

<210> 5
<211> 29
<212> DNA
<213> Artificial Sequence

<220>
<223> Designed oligonucleotide primer for PCR

<400> 5
acctatgagc agcaatggat agagtctat 29

<210> 6
<211> 26
<212> DNA
<213> Artificial Sequence

<220>
<223> Designed oligonucleotide primer for PCR

<400> 6
atcatcacaa caggaaagta ataaaa 26

<210> 7

<211> 24
<212> DNA
<213> Artificial Sequence

<220>
<223> Designed oligonucleotide primer for PCR

<400> 7
attgtgcatc cttcgaacgt ccca 24

<210> 8
<211> 23
<212> DNA
<213> Artificial Sequence

<220>
<223> Designed oligonucleotide primer for PCR

<400> 8
tggtgccctc ttctggtgtg tct 23

<210> 9
<211> 30
<212> DNA
<213> Artificial Sequence

<220>
<223> Designed oligonucleotide primer for PCR

<400> 9
atgccatata cttgagtcac agtttgtgaa 30

<210> 10
<211> 26
<212> DNA
<213> Artificial Sequence

<220>
<223> Designed oligonucleotide primer for PCR

<400> 10
ctcccgagcc ttcagggatc ttcttt 26

<210> 11
<211> 30
<212> DNA
<213> Artificial Sequence

<220>
<223> Designed oligonucleotide primer for PCR

<400> 11
agactgctcc ggttaccgta aatactgcct 30